# EUROPEAN PATENT SPECIFICATION

(11) **EP 3 784 803 B1**
(45) Date of publication and mention of the grant of the patent: **11.02.2026**
(21) Application number: 19718750.3
(22) Date of filing: 25.04.2019
(51) Int. Cl.: C12Q 1/6886, G01N 33/574, C07K 14/74

(54) **HLA-J AND MEDICAL/DIAGNOSTIC USES THEREOF**
HLA-J UND MEDIZINISCHE/DIAGNOSTISCHE VERWENDUNGEN DAVON
HLA-J ET UTILISATIONS DE DIAGNOSTIC/MÉDICALES

(30) Priority: 26.04.2018 EP 18169662
(43) Date of publication of application: 03.03.2021
(73) Proprietor: Intellexon GmbH, 82343 Pöcking (DE)
(72) Inventor: WÜRFEL, Wolfgang, 85235 Odelzhausen (DE); WIRTZ, Ralph Markus, 50935 Köln (DE); WINTERHALTER, Christoph, 82343 Pöcking (DE); WÜRFEL, Franziska, 85235 Odelzhausen (DE)
(74) Representative: Vossius & Partner Patentanwälte Rechtsanwälte mbB
(86) International application number: PCT/EP2019/060606
(87) International publication number: WO 2019/207039

(56) References cited:
- EP-A1- 2 561 890
- WO-A2-2008/021290
- US-B2- 9 353 416
- DATABASE Genpept [online] 18 March 2009 (2009-03-18), "HLA-J protein [Homo sapiens]", XP093274059, retrieved from https://www.ncbi.nlm.nih.gov/protein/AAH89454.1?report=genpept Database accession no. AAH89454
- DATABASE UniProt [online] 1 November 1996 (1996-11-01), "SubName: Full=MHC class I HLA-J antigen {ECO:0000313|EMBL:AAA66955.1}; Flags: Fragment;", XP002785814, retrieved from EBI accession no. UNIPROT:Q30175 Database accession no. Q30175
- DATABASE EMBL [online] 3 February 2005 (2005-02-03), "Homo sapiens major histocompatibility complex, class I, J (pseudogene), mRNA (cDNA clone IMAGE:30563285).", XP002785815, retrieved from EBI accession no. EM_STD:BC089454 Database accession no. BC089454
- DATABASE EMBL [online] 7 December 1991 (1991-12-07), "Human MHC class I HLA-J gene, exons 1-8 and complete cds.", XP002785816, retrieved from EBI accession no. EMBL:M80469 Database accession no. M80469
- MESSER ET AL: "HLA-J, a second inactivated class I HLA gene related to HLA-G and HLA-A", THE JOURNAL OF IMMUNOLOGY, vol. 148, 15 June 1992 (1992-06-15), pages 4043 - 4053, XP055516437, Retrieved from the Internet <URL:http://www.jimmunol.org/content/148/12/4043.full-text.pdf>
- DATABASE UniProt [online] 3 March 2009 (2009-03-03), XP002793065, retrieved from https://www.uniprot.org/uniprot/Q5FWE1.txt?version=25 Database accession no. Q5FWE1

## Description

The present invention is defined by the claims and accordingy *inter alia* relates to a nucleic acid molecule, a vector, a host cell, or a protein or peptide, or combinations thereof for use as an immunosuppressant medicament or as a tumor vaccine, wherein (I) the nucleic acid molecule is (a) encoding a polypeptide comprising or consisting of the amino acid sequence of SEQ ID NO: 1, (b) consisting of the nucleotide sequence of SEQ ID NO: 2, (c) encoding a polypeptide which at least 80% identical, preferably at least 90% identical, and most preferred at least 95% identical to the amino acid sequence of SEQ ID NO: 1; (d) consisting of a nucleotide sequence which at least 80% identical, preferably at least 90% identical, and most preferred at least 95% identical to the nucleotide sequence of SEQ ID NO: 2; (e) consisting of a nucleotide sequence which is degenerate with respect to the nucleic acid molecule of (d); (f) a fragment of the nucleic acid molecule of any one of (a) to (e), said fragment comprising at least 350 nucleotides, preferably at least 450 nucleotides, and most preferably at least 600 nucleotides; or (g) corresponding to the nucleic acid molecule of any one of (a) to (f), wherein T is replaced by U; (II) the vector comprises the nucleic acid molecule of (I); (III) the host cell is transformed, transduced or transfected with the vector of (II); and (IV) the protein or peptide being encoded by the nucleic acid molecule of (I).

The human leukocyte antigen (HLA) system or complex is a gene complex encoding the major histocompatibility complex (MHC) proteins in humans. These cell-surface proteins are responsible for the regulation of the immune system in humans. The HLA gene complex resides on a 3 Mbp stretch within chromosome 6p21. Genes in this complex are categorized into three basic groups: class I, class II, and class III.

Humans have three main MHC class I genes, known as HLA-A, HLA-B, and HLA-C. The proteins produced from these genes are present on the surface of almost all cells. On the cell surface, these proteins are bound to protein fragments (peptides) that have been exported from within the cell. MHC class I proteins display these peptides to the immune system. If the immune system recognizes the peptides as foreign (such as viral or bacterial peptides), it responds by triggering the infected cell to self-destruction.

There are six main MHC class II genes in humans: HLA-DPA1, HLA-DPB1, HLA-DQA1, HLA-DQB1, HLA-DRA, and HLA-DRB1. MHC class II genes provide instructions for making proteins that are present almost exclusively on the surface of certain immune system cells. Like MHC class I proteins, these proteins display peptides to the immune system.

The proteins produced from MHC class III genes have somewhat different functions; they are involved in inflammation and other immune system activities. The functions of some MHC genes are unknown.

HLA genes have many possible variations, allowing each person's immune system to react to a wide range of foreign invaders. Some HLA genes have hundreds of identified versions (alleles), each of which is given a particular number (such as HLA-B27). Closely related alleles are categorized together; for example, at least 40 very similar alleles are subtypes of HLA-B27. These subtypes are designated as HLA-B*2701 to HLA-B*2743.

More than 100 diseases have been associated with different alleles of HLA genes. For example, the HLA-B27 allele increases the risk of developing an inflammatory joint disease called ankylosing spondylitis. Many other disorders involving abnormal immune function and some forms of cancer have also been associated with specific HLA alleles. However, it is often unclear what role HLA genes play in the risk of developing these diseases.

Next to the three main MHC class I genes the non-classical MHC class I molecules HLA-E, HLA-F HLA-G are encoded by the HLA class I region. The overexpression of HLA-G, -E, and -F is a common finding across a variety of malignancies (Kochan et al., Oncoimmunology. 2013 Nov 1; 2(11): e26491.). HLA-G and HLA-E were reported as being cancer biomarkers and also as being positively correlated with poor clinical outcome of cancer.

The HLA class I region was furthermore reported to include class I pseudogenes (Hughes, Mol Biol Evol. 1995 Mar; 12(2):247-58) as well as gene fragments. For instance, HLA-H, J, K and L are classified as class I pseudogenes and HLA-N, S and X are classified as gene fragments. In particular, it was reported by Messer et al., J Immunol. 1992 Jun 15; 148(12):4043-53 that HLA-J is a pseudogene, due to deleterious mutations that produce a translation termination either in exon 2 or exon 4.

US 9,353,416 B2 discusses that HLA-J can be used as a marker in the post-operative prognosis of patients afflicted with breast cancer. More specficically, HLA-J, together with various other markers, provides for a good prognosis when overexpressed. As regards the nature of the HLA-J sequence it is assumed in US 9,353,416 B2 that the HLA-J sequence as reported by Messer et al., J Immunol. 1992 Jun 15; 148(12):4043-53 is correct. Moreover and in line with the assumption that HLA-J is a marker for a good prognosis, US 9,353,416 B2 is silent about the potential use of HLA-J as a tumor vaccine.

Hence, human leukocyte antigen (HLA) genes have a long research history as important targets in biomedical science and treatment. However, in view of the clinical importance of the HLA system there is still a need to focus research on the HLA genes and in particular to identify further targets for biomedical science and treatment based on the HLA system. This need is addressed by the present invention. In connection with the present invention it was surprisingly found that HLA-J is not a pseudogene but comprises a functional open reading frame encoding a HLA-J protein and that HLA J is a target for the treatment and detection of diseases, in particular tumors.

Hence, the present invention relates in a first aspect to a nucleic acid molecule, a vector, a host cell, or a protein or peptide, or combinations thereof for use as an immunosuppressant medicament or as a tumor vaccine, wherein (I) the nucleic acid molecule is (a) encoding a polypeptide comprising or consisting of the amino acid sequence of SEQ ID NO: 1, (b) consisting of the nucleotide sequence of SEQ ID NO: 2, (c) encoding a polypeptide which is at least 80% identical, preferably at least 90% identical, and most preferred at least 95% identical to the amino acid sequence of SEQ ID NO: 1; (d) consisting of a nucleotide sequence which is at least 80% identical, preferably at least 90% identical, and most preferred at least 95% identical to the nucleotide sequence of SEQ ID NO: 2; provided; (e) consisting of a nucleotide sequence which is degenerate with respect to the nucleic acid molecule of (d); (f) a fragment of the nucleic acid molecule of any one of (a) to (e), said fragment comprising at least 350 nucleotides, preferably at least 450 nucleotides, and most preferably at least 600 nucleotides; or (g) corresponding to the nucleic acid molecule of any one of (a) to (f), wherein T is replaced by U; (II) the vector comprises the nucleic acid molecule of (I); (III) the host cell is transformed, transduced or transfected with the vector of (II); and (IV) the protein or peptide being encoded by the nucleic acid molecule of (I).

The term "nucleic acid molecule" in accordance with the present invention includes DNA, such as cDNA or double or single stranded genomic DNA and RNA. In this regard, "DNA" (deoxyribonucleic acid) means any chain or sequence of the chemical building blocks adenine (A), guanine (G), cytosine (C) and thymine (T), called nucleotide bases, that are linked together on a deoxyribose sugar backbone. DNA can have one strand of nucleotide bases, or two complimentary strands which may form a double helix structure. "RNA" (ribonucleic acid) means any chain or sequence of the chemical building blocks adenine (A), guanine (G), cytosine (C) and uracil (U), called nucleotide bases, that are linked together on a ribose sugar backbone. RNA typically has one strand of nucleotide bases, such as mRNA. Included are also single- and double-stranded hybrids molecules, i.e., DNA-DNA, DNA-RNA and RNA-RNA. The nucleic acid molecule may also be modified by many means known in the art. Non-limiting examples of such modifications include methylation, "caps", substitution of one or more of the naturally occurring nucleotides with an analog, and internucleotide modifications such as, for example, those with uncharged linkages (e.g., methyl phosphonates, phosphotriesters, phosphoroamidates, carbamates, etc.) and with charged linkages (e.g., phosphorothioates, phosphorodithioates, etc.). Nucleic acid molecules, in the following also referred as polynucleotides, may contain one or more additional covalently linked moieties, such as, for example, proteins (e.g., nucleases, toxins, antibodies, signal peptides, poly-L-lysine, etc.), intercalators (e.g., acridine, psoralen, etc.), chelators (e.g., metals, radioactive metals, iron, oxidative metals, etc.), and alkylators. The polynucleotides may be derivatized by formation of a methyl or ethyl phosphotriester or an alkyl phosphoramidate linkage. Further included are nucleic acid mimicking molecules known in the art such as synthetic or semi-synthetic derivatives of DNA or RNA and mixed polymers. Such nucleic acid mimicking molecules or nucleic acid derivatives according to the invention include phosphorothioate nucleic acid, phosphoramidate nucleic acid, 2'-O-methoxyethyl ribonucleic acid, morpholino nucleic acid, hexitol nucleic acid (HNA), peptide nucleic acid (PNA) and locked nucleic acid (LNA) (see Braasch and Corey, Chem Biol 2001, 8: 1). LNA is an RNA derivative in which the ribose ring is constrained by a methylene linkage between the 2'-oxygen and the 4'-carbon. Also included are nucleic acids containing modified bases, for example thio-uracil, thio-guanine and fluoro-uracil. A nucleic acid molecule typically carries genetic information, including the information used by cellular machinery to make proteins and/or polypeptides. The nucleic acid molecule of the invention may additionally comprise promoters, enhancers, response elements, signal sequences, polyadenylation sequences, introns, 5'- and 3'- non-coding regions, and the like.

The nucleic acid molecule according to the invention encodes a polypeptide or fragment thereof which is derived from the HLA-J protein of SEQ ID NO: 1. It is therefore preferred that the nucleic acid molecule of the invention is genomic DNA or mRNA. In the case of mRNA, the nucleic acid molecule may in addition comprise a poly-A tail.

The term "protein" as used herein interchangeably with the term "polypeptide" describes linear molecular chains of amino acids, including single chain proteins or their fragments, containing at least 50 amino acids. The term "peptide" as used herein describes a group of molecules consisting of up to 49 amino acids, whereas the term "polypeptide" (also referred to as "protein") as used herein describes a group of molecules consisting of at least 50 amino acids. The term "peptide" as used herein describes a group of molecules consisting with increased preference of at least 15 amino acids, at least 20 amino acids at least 25 amino acids, and at least 40 amino acids. The group of peptides and polypeptides are referred to together by using the term "(poly)peptide". (Poly)peptides may further form oligomers consisting of at least two identical or different molecules. The corresponding higher order structures of such multimers are, correspondingly, termed homo- or heterodimers, homo- or heterotrimers etc.. The HLA-J protein of SEQ ID NO: 1 comprises cysteins at positions 139, 175 and 189 and thus potential dimerization sites. Furthermore, peptidomimetics of such proteins/(poly)peptides where amino acid(s) and/or peptide bond(s) have been replaced by functional analogues are also encompassed by the invention. Such functional analogues include all known amino acids other than the 20 gene-encoded amino acids, such as selenocysteine. The terms "(poly)peptide" and "protein" also refer to naturally modified (poly)peptides and proteins where the modification is effected e.g. by glycosylation, acetylation, phosphorylation and similar modifications which are well known in the art.

In accordance with the present invention, the term "percent (%) sequence identity" describes the number of matches ("hits") of identical nucleotides/amino acids of two or more aligned nucleic acid or amino acid sequences as compared to the number of nucleotides or amino acid residues making up the overall length of the template nucleic acid or amino acid sequences. In other terms, using an alignment, for two or more sequences or subsequences the percentage of amino acid residues or nucleotides that are the same (e.g. 80%, 85%, 90% or 95% identity) may be determined, when the (sub)sequences are compared and aligned for maximum correspondence over a window of comparison, or over a designated region as measured using a sequence comparison algorithm as known in the art, or when manually aligned and visually inspected. This definition also applies to the complement of any sequence to be aligned.

Nucleotide and amino acid sequence analysis and alignment in connection with the present invention are preferably carried out using the NCBI BLAST algorithm (Stephen F. Altschul, Thomas L. Madden, Alejandro A. Schäffer, Jinghui Zhang, Zheng Zhang, Webb Miller, and David J. Lipman (1997), Nucleic Acids Res. 25:3389-3402). BLAST can be used for nucleotide sequences (nucleotide BLAST) and amino acid sequences (protein BLAST). The skilled person is aware of additional suitable programs to align nucleic acid sequences.

As defined herein, sequence identities of at least 80% identical, preferably at least 90% identical, and most preferred at least 95% are envisaged by the invention. However, also envisaged by the invention are with increasing preference sequence identities of at least 97.5%, at least 98.5%, at least 99%, at least 99.5%, at least 99.8%, and 100%.

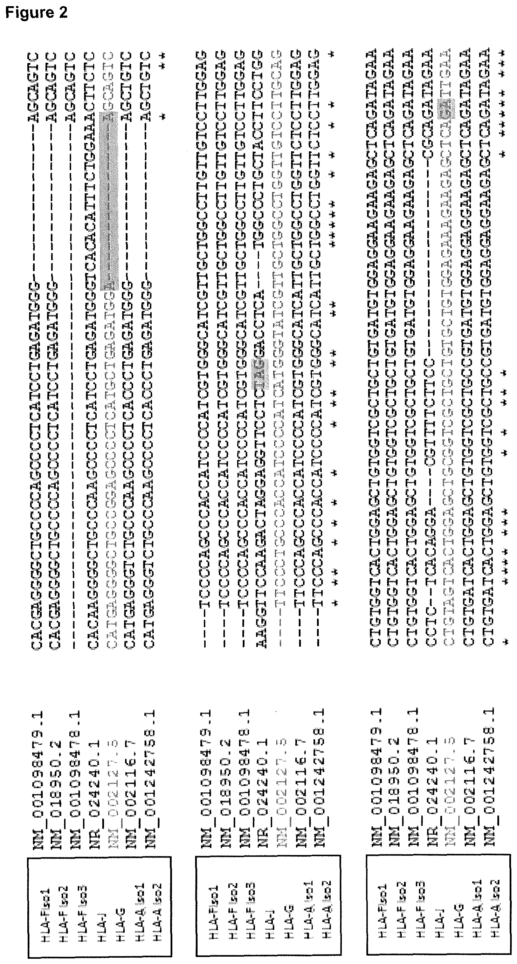

Moreover, it is preferred that within the nucleotide sequences having at least 80% identity or any one of the preferred higher identities with the nucleotide sequence of SEQ ID NO: 2 the 16 nucleotides "TCACACATTTCTGGAA" (SEQ ID NO: 3) of SEQ ID NO: 2 are present and remain unchanged. As is evident from Figure 2, SEQ ID NO: 3 corresponds to an insertion mutation of 16 nucleotides which can only be found in the HLA-J nucleic acid molecule of SEQ ID NO: 2 but cannot be found in the nucleic acid molecules of other HLA classes. The 16 nucleotides insertion is thus unique for the HLA-J nucleic acid molecule. Moreover, the nucleotides insertion results in a frame shift, so that the entire nucleic acid sequences from the insertion until the 3'-end of SEQ ID NO: 2 - except the stop codon "TAG" - encodes a unique peptide. It is accordingly more preferred that within the nucleotide sequences having at least 80% identity or any one of the preferred higher identities with the nucleotide sequence of SEQ ID NO: 2 the nucleotides "TCACACATTTCTGGAAACTTCTCAAGG TTCCAAGACTAGGAGGTTCCTC" (SEQ ID NO: 4) of SEQ ID NO: 2 are present and remain unchanged. The amino acids being encoded by SEQ ID NO: 4 are "HTFLETSQGSKTRRFL" (SEQ ID NO: 5). It is therefore more preferred that the nucleotide sequences having at least 80% identity or any one of the preferred higher identities with the nucleotide sequence of SEQ ID NO: 2 encode an amino acid sequence comprising SEQ ID NO: 5, preferably at the C-terminus.

MHC class I molecules are generally comprised of two chains: a MHC alpha chain (heavy chain), and a beta2-microglobulin chain (light chain). Only the alpha chain spans the membrane. The alpha chain has three extracellular domains (being designated as alpha 1, 2 and 3 and with alpha 1 being at the N-terminus). It is believed that the alpha chain domains alpha 1 and alpha 3 of HLA-J predominately determine the immunosuppressive capability of HLA-J, wherein the domain alpha 3 is most important. The nucleotide sequences of SEQ ID NOs 6 and 7 encode the domains alpha 1 and alpha 3 of HLA-J, respectively. The amino acid sequences of SEQ ID NOs 8 and 9 are the amino acid sequences of the domains alpha 1 and alpha 3 of HLA-J, respectively. It is therefore preferred that the nucleotide sequences having at least 80% identity or any one of the preferred higher identities with the nucleotide sequence of SEQ ID NO: 2 comprise a nucleotide sequence being with increased preference at least 97.5%, at least 98.5%, at least 99%, at least 99.5%, at least 99.8%, and 100% identical to SEQ ID NO: 7. It is also preferred that the nucleotide sequences having at least 80% identity or any one of the preferred higher identities with the nucleotide sequence of SEQ ID NO: 2 encode an amino acid sequence being with increased preference at least 97.5%, at least 98.5%, at least 99%, at least 99.5%, at least 99.8%, and 100% identical to SEQ ID NO: 9. It is more preferred that the nucleotide sequences having at least 80% identity or any one of the preferred higher identities with the nucleotide sequence of SEQ ID NO: 2 comprise a nucleotide sequence being with increased preference at least 97.5%, at least 98.5%, at least 99%, at least 99.5%, at least 99.8%, and 100% identical to SEQ ID NO: 7 and/or comprise a nucleotide sequence being with increased preference at least 97.5%, at least 98.5%, at least 99%, at least 99.5%, at least 99.8%, and 100% identical to SEQ ID NO: 6. It is also more preferred that the nucleotide sequences having at least 80% identity or any one of the preferred higher identities with the nucleotide sequence of SEQ ID NO: 2 encode an amino acid sequence being with increased preference at least 97.5%, at least 98.5%, at least 99%, at least 99.5%, at least 99.8%, and 100% identical to SEQ ID NO: 9 and/or encode an amino acid sequence being with increased preference at least 97.5%, at least 98.5%, at least 99%, at least 99.5%, at least 99.8%, and 100% identical to SEQ ID NO: 8.

It is most preferred that the nucleotide sequences having at least 80% identity or any one of the preferred higher identities with the nucleotide sequence of SEQ ID NO: 2 comprise two or all three of: (i) the nucleotide sequence of SEQ ID NO: 3, preferably of SEQ ID NO: 4, wherein SEQ ID NO: 4 is preferably at the 3'-end (ii) a nucleotide sequence being with increased preference at least 97.5%, at least 98.5%, at least 99%, at least 99.5%, at least 99.8%, and 100% identical to SEQ ID NO: 7 and (iii) nucleotide sequence being with increased preference at least 97.5%, at least 98.5%, at least 99%, at least 99.5%, at least 99.8%, and 100% identical to SEQ ID NO: 6. In the case of two of (i) to (iii) the two are preferably (i) and (ii).

The term "degenerate" in accordance with the present invention refers to the degeneracy of the genetic code. Degeneracy results because a triplet code designates 20 amino acids and a stop codon and because four bases exist which are utilized to encode genetic information, triplet codons are required to produce at least 21 different codes. The possible 4³ possibilities for bases in triplets give 64 possible codons, meaning that some degeneracy must exist. As a result, some amino acids are encoded by more than one triplet, i.e. by up to six. The degeneracy mostly arises from alterations in the third position in a triplet. This means that nucleic acid molecules having a different nucleotide sequence than that specified above, but still encoding the same polypeptide lie within the scope of the present invention. With regard to the first aspect of the invention, the skilled person thus understands that "(e) consisting of a nucleotide sequence which is degenerate with respect to the nucleic acid molecule of (d)" as recited in item (e) designates a nucleic acid molecule which encodes the same amino acid sequence as the nucleic acid molecule according to item (d). This amino acid sequence is either the amino acid sequence of SEQ ID NO: 1 or derived therefrom, said latter amino acid sequence being identical to SEQ ID NO: 1 at least to the extent as required and implied by the sequence identity values recited in item (d) of the main embodiment.

Fragments of the nucleic acid molecule of any one of (a) to (f) according to the present invention comprise at least 150 nucleotides. In this regard, it is preferred with increasing preference that the fragments according the present invention are polynucleotides of at least 350, at least 400, at least 450, at least 500, at least 550, at least 600, or at least 650 nucleotides and most preferred that the fragment is a fragment only lacking the 5'- ATP start codon and/or the 3'-TAG stop codon. Moreover, it is preferred that the fragment comprises SEQ ID NO: 3, preferably SEQ ID NO: 4 and most preferably a nucleotide sequence encoding SEQ ID NO: 5. The nucleotide sequence encoding SEQ ID NO: 5 is preferably found at the 3'-end of the fragment either followed by no further nucleotide(s) or by a stop codon. It is it likewise preferred that the fragment comprises a nucleotide sequence being with increased preference at least 97.5%, at least 98.5%, at least 99%, at least 99.5%, at least 99.8%, and 100% identical to SEQ ID NO: 7 or encodes an amino acid sequence being with increased preference at least 97.5%, at least 98.5%, at least 99%, at least 99.5%, at least 99.8%, and 100% identical to SEQ ID NO: 9. It is more preferred that the fragment comprises a nucleotide sequence being with increased preference at least 97.5%, at least 98.5%, at least 99%, at least 99.5%, at least 99.8%, and 100% identical to SEQ ID NO: 7 and/or a nucleotide sequence being with increased preference at least 97.5%, at least 98.5%, at least 99%, at least 99.5%, at least 99.8%, and 100% identical to SEQ ID NO: 6. Similarly, it is more preferred that the fragment encodes an amino acid sequence being with increased preference at least 97.5%, at least 98.5%, at least 99%, at least 99.5%, at least 99.8%, and 100% identical to SEQ ID NO: 9 and/or encodes an amino acid sequence being with increased preference at least 97.5%, at least 98.5%, at least 99%, at least 99.5%, at least 99.8%, and 100% identical to SEQ ID NO: 8. It is most preferred that the fragment comprises two or all three of: (i) the nucleotide sequence of SEQ ID NO: 3, preferably of SEQ ID NO: 4, wherein SEQ ID NO: 4 is preferably at the 3'-end (ii) a nucleotide sequence being with increased preference at least 97.5%, at least 98.5%, at least 99%, at least 99.5%, at least 99.8%, and 100% identical to SEQ ID NO: 7 and (iii) nucleotide sequence being with increased preference at least 97.5%, at least 98.5%, at least 99%, at least 99.5%, at least 99.8%, and 100% identical to SEQ ID NO: 6. In the case of two of (i) to (iii) the two are preferably (i) and (ii).

It was reported by Messer et al. in 1992 (J Immunol. 1992 Jun 15; 148(12):4043-53.) that HLA-J is a pseudogene, due to deleterious mutations that produce a translation termination either in exon 2 or exon 4. Since then the firm belief that HLA-J is a pseudogene persisted in the art. As is evident from the examples herein below, it was only now surprisingly found that the HLA-J nucleotide sequence comprises a functional open reading frame (designated "open reading frame 3" or "ORF3" herein) for which it can safely be assumed that it encodes a HLA-J protein. The sequence of the functional open reading frame is shown in SEQ ID NO: 2 and the HLA-J protein encoded thereby is shown in SEQ ID NO: 1. Hence, after more than 25 years it was unexpectedly revealed that HLA-J is not pseudogene but a functional gene; see Example 1.

It is of further note that ORF3 comprises an insertion of 16 nucleotides which is unique for HLA-J. No other HLA comprises a similar sequence. Due to this insertion HLA-J lacks a homolog of exon 4 of HLA-G, which exon codes for the alpha domain structure 2. The alpha 2 like domain is important for peptide presentation. Even more importantly, the insertions leads to a unique 3-end of the ORF3 which encodes the peptide "HTFLETSQGSKTRRFL(-Stop)" (SEQ ID NO: 5). Due to the multiple charged amino acids and aromatic amino acids within this peptide it can be safely excluded that the HLA-J protein comprises a transmembrane spanning region or anchor. The newly identified HLA-J protein is therefore a secreted protein with features similar to HLA-G but lacking the capability of peptide presentation due to the absence of the alpha 2 like domain; see Example 1.

In accordance with a preferred embodiment of the first aspect of the invention the nucleic acid molecule is fused to a heterologous nucleotide sequence, preferably operably linked to a heterologous promoter.

The heterologous nucleotide sequence can either be directly or indirectly fused to the nucleic acid molecule of the invention. In case of an indirect fusion preferably nucleotide sequences encoding a peptide linker are used for the fusion, such that a GS-linker (e.g. Gly-Gly-Gly-Gly-Ser)n (SEQ ID NO: 54), wherein n is 1 to 3).

As used herein, a heterologous nucleotide sequence is a sequence that cannot be found in nature fused to the nucleotide sequence of SEQ ID NO: 2. Noting that SEQ ID NO: 2 is from human, it is preferred that the heterologous nucleotide sequence is also derived from human.

Accordingly, a heterologous promoter is a promoter that cannot be found in nature operably linked to the nucleotide sequence of SEQ ID NO: 2. The heterologous promoter is preferably from human.

A promoter is a nucleic acid sequence that initiates transcription of a particular gene, said gene being in accordance with the invention derived from the HLA-J gene of SEQ ID NO: 2 or being SEQ ID NO: 2. In this connection "operably linked" shall mean that the heterologous promoter is fused to the nucleic acid molecule of the invention, so that via the promoter the transcription of the nucleic acid molecule of the invention can be initiated, for example, in prokaryotes or eukaryotic cells. The heterologous promoter can be a constitutively active promoter, a tissue-specific or development-stage-specific promoter, an inducible promoter, or a synthetic promoter. Constitutive promoters direct expression in virtually all tissues and are largely, if not entirely, independent of environmental and developmental factors. As their expression is normally not conditioned by endogenous factors, constitutive promoters are usually active across species and even across kingdoms. Tissue-specific or development-stage-specific promoters direct the expression of a gene in specific tissue(s) or at certain stages of development. The activity of inducible promoters is induced by the presence or absence of biotic or abiotic factors. Inducible promoters are a very powerful tool in genetic engineering because the expression of genes operably linked to them can be turned on or off as needed. Synthetic promoters are constructed by bringing together the primary elements of a promoter region from diverse origins.

Non-limiting examples of heterologous promoters which are used in the art in order to express genes heterologously are SV40, CMV, HSV, UBC, EF1A, PGK, Vlambda1, RSV and CAGG (for mammalian systems); COPIA and ACT5C (for Drosophila systems) and GALA1, GAL10, GAL7, GAL2 (for yeast systems) and can also be employed in connection with the present invention.

Alternatively or in addition, the heterologous nucleic acid sequence may be a coding sequence such that the nucleic acid sequence of the invention gives rise to a fusion protein. Such fusion proteins are discussed in more detail herein below.

If the nucleic acid molecule is not fused to a heterologous promoter, then for expression purposes it is fused to its own promoter.

The term "vector" in accordance with the invention means preferably a plasmid, cosmid, virus, bacteriophage or another vector used e.g. conventionally in genetic engineering which carries the nucleic acid molecule of the invention. The nucleic acid molecule of the invention may, for example, be inserted into several commercially available vectors. Non-limiting examples include prokaryotic plasmid vectors, such as of the pUC-series, pBluescript (Stratagene), the pET-series of expression vectors (Novagen) or pCRTOPO (Invitrogen) and vectors compatible with an expression in mammalian cells like pREP (Invitrogen), pcDNA3 (Invitrogen), pCEP4 (Invitrogen), pMC1neo (Stratagene), pXT1 (Stratagene), pSG5 (Stratagene), EBO-pSV2neo, pBPV-1, pdBPVMMTneo, pRSVgpt, pRSVneo, pSV2-dhfr, plZD35, pLXIN, pSIR (Clontech), pIRES-EGFP (Clontech), pEAK-10 (Edge Biosystems) pTriEx-Hygro (Novagen) and pCINeo (Promega). Examples for plasmid vectors suitable for Pichia pastoris comprise e.g. the plasmids pAO815, pPIC9K and pPIC3.5K (all Invitrogen).

The nucleic acid molecules inserted into the vector can e.g. be synthesized by standard methods, or isolated from natural sources. Ligation of the coding sequences to transcriptional regulatory elements and/or to other amino acid encoding sequences can also be carried out using established methods. Transcriptional regulatory elements (parts of an expression cassette) ensuring expression in prokaryotes or eukaryotic cells are well known to those skilled in the art. These elements comprise regulatory sequences ensuring the initiation of transcription (e. g., translation initiation codon, promoters, such as naturally-associated or heterologous promoters and/or insulators; see above), internal ribosomal entry sites (IRES) (Owens, Proc. Natl. Acad. Sci. USA 98 (2001), 1471-1476) and optionally poly-A signals ensuring termination of transcription and stabilization of the transcript. Additional regulatory elements may include transcriptional as well as translational enhancers. Preferably, the polynucleotide encoding the polypeptide/protein or fusion protein of the invention is operatively linked to such expression control sequences allowing expression in prokaryotes or eukaryotic cells. The vector may further comprise nucleic acid sequences encoding secretion signals as further regulatory elements. Such sequences are well known to the person skilled in the art. Furthermore, depending on the expression system used, leader sequences capable of directing the expressed polypeptide to a cellular compartment may be added to the coding sequence of the polynucleotide of the invention. Such leader sequences are well known in the art.

Furthermore, it is preferred that the vector comprises a selectable marker. Examples of selectable markers include genes encoding resistance to neomycin, ampicillin, hygromycine, and kanamycin. Specifically-designed vectors allow the shuttling of DNA between different hosts, such as bacteria-fungal cells or bacteria-animal cells (e. g. the Gateway system available at Invitrogen). An expression vector according to this invention is capable of directing the replication, and the expression, of the polynucleotide and encoded peptide or fusion protein of this invention. Apart from introduction via vectors such as phage vectors or viral vectors (e.g. adenoviral, retroviral), the nucleic acid molecules as described herein above may be designed for direct introduction or for introduction via liposomes into a cell. Additionally, baculoviral systems or systems based on vaccinia virus or Semliki Forest virus can be used as eukaryotic expression systems for the nucleic acid molecules of the invention.

The term "host cell" means any cell of any organism that is selected, modified, transformed, grown, or used or manipulated in any way, for the production of the protein or peptide or fusion protein of the invention by the cell.

The host cell of the invention is typically produced by introducing the nucleic acid molecule or vector(s) of the invention into the host cell which upon its/their presence mediates the expression of the nucleic acid molecule of the invention encoding the protein or peptide or fusion protein of the invention. The host from which the host cell is derived or isolated may be any prokaryote or eukaryotic cell or organism, preferably with the exception of human embryonic stem cells that have been derived directly by destruction of a human embryo.

Suitable prokaryotes (bacteria) useful as hosts for the invention are, for example, those generally used for cloning and/or expression like *E. coli* (e.g., *E coli* strains BL21, HB101, DH5a, XL1 Blue, Y1090 and JM101), *Salmonella typhimurium, Serratia marcescens, Burkholderia glumae, Pseudomonas putida, Pseudomonas fluorescens, Pseudomonas stutzeri, Streptomyces lividans, Lactococcus lactis, Mycobacterium smegmatis, Streptomyces coelicolor* or *Bacillus subtilis.* Appropriate culture mediums and conditions for the above-described host cells are well known in the art.

A suitable eukaryotic host cell may be a vertebrate cell, an insect cell, a fungal/yeast cell, a nematode cell or a plant cell. The fungal/yeast cell may a *Saccharomyces cerevisiae* cell, *Pichia pastoris* cell or an *Aspergillus* cell. Preferred examples for host cell to be genetically engineered with the nucleic acid molecule or the vector(s) of the invention is a cell of yeast, *E. coli* and/or a species of the genus *Bacillus* (e.g., *B. subtilis*)*.* In one preferred embodiment the host cell is a yeast cell (e.g. *S. cerevisiae*)*.*

In a different preferred embodiment the host cell is a mammalian host cell, such as a Chinese Hamster Ovary (CHO) cell, mouse myeloma lymphoblastoid, human embryonic kidney cell (HEK-293), human embryonic retinal cell (Crucell's Per.C6), or human amniocyte cell (Glycotope and CEVEC). The cells are frequently used in the art to produce recombinant proteins. CHO cells are the most commonly used mammalian host cells for industrial production of recombinant protein therapeutics for humans.

The terms "protein" and "peptide" and preferred embodiments thereof have been defined herein above in connection with the first aspect of the invention. The peptide is preferably at least 80%, preferably at least 90% and most preferably at least 95% identical to a subsequence of SEQ ID NO: 1. Most preferably the peptide comprises or consists of SEQ ID NO: 5.

The protein or peptide of the invention may be generated by molecular cloning techniques well known in the art. Recombinant expression can be accomplished, for example, by using vectors and host cells as described herein above.

The detection of the protein or peptide of the invention in blood and tissue samples from cancer patients is shown in Example 4.

According to a preferred embodiment, the protein or peptide of the invention is a fusion protein.

A "fusion protein" according to the present invention contains at least one additional heterologous amino acid sequence. Often, but not necessarily, these additional sequences will be located at the N- or C-terminal end of the (poly)peptide. It may e.g. be convenient to initially express the polypeptide as a fusion protein from which the additional amino acid residues can be removed, e.g. by a proteinase capable of specifically trimming the fusion protein and releasing the (poly)peptide of the present invention. The amino acid sequence compound can either be directly or indirectly fused to the nucleic acid molecule of the invention. In case of an indirect fusion generally a peptide linker may be used for the fusion, such that a GS-linker (e.g. Gly-Gly-Gly-Gly-Ser)n (SEQ ID NO: 54), wherein n is 1 to 3).

Those at least one additional heterologous amino acid sequence of said fusion proteins includes amino acid sequences which confer desired properties such as modified/enhanced stability, modified/enhanced solubility and/or the ability of targeting one or more specific cell types. For example, fusion proteins with antibodies. The term antibody is further defined herein below and *inter alia* comprises antibody fragments and derivatives. The antibody may be, for example, specific for cell surface markers or may be an antigen-recognizing fragment of said antibodies. The protein or peptide of the invention can be fused to the N-terminus or C-terminus of the light and/or heavy chain(s) of an antibody. The protein or peptide of the invention is preferably fused to the N-terminus of the light and/or heavy chain(s) of an antibody, so that the Fc part of the antibody is free to bind to Fc-receptors.

The fusion protein may also comprise protein domains known to function in signal transduction and/or known to be involved in protein-protein interaction. Examples for such domains are Ankyrin repeats; arm, Bcl-homology, Bromo, CARD, CH, Chr, C1, C2, DD, DED, DH, EFh, ENTH, F-box, FHA, FYVE, GEL, GYF, hect, LIM, MH2, PDZ, PB1, PH, PTB, PX, RGS, RING, SAM, SC, SH2, SH3, SOCS, START, TIR, TPR, TRAF, tsnare, Tubby, UBA, VHS, W, WW, and 14-3-3 domains. Further information about these and other protein domains is available from the databases InterPro (http://www.ebi.ac.uk/interpro/, Mulder et al., 2003, Nucl. Acids. Res. 31: 315-318), Pfam (http://www.sanger.ac.uk/Software/Pfam/, Bateman et al., 2002, Nucleic Acids Research 30(1): 276-280) and SMART (http://smart.embl-heidelberg.de/, Letunic et al., 2002, Nucleic Acids Res. 30(1), 242-244).

The at least one additional heterologous amino acid sequence of the fusion protein according to the present invention may comprise or consist of (a) a cytokine, (b) a chemokine, (c) a pro-coagulant factor, (d) a proteinaceous toxic compound, and/or (e) an enzyme for pro-drug activation.

The cytokine is preferably selected from the group consisting of IL-2, IL-12, TNF-alpha, IFN alpha, IFN beta, IFN gamma, IL-10, IL-15, IL-24, GM-CSF, IL-3, IL-4, IL-5, IL-6, IL-7, IL-9, IL-11, IL-13, LIF, CD80, B70, TNF beta, LT-beta, CD-40 ligand, Fas-ligand, TGF-beta, IL-1alpha and IL-1beta. As it is well-known in the art, cytokines may favour a pro-inflammatory or an anti-inflammatory response of the immune system. Thus, depending on the disease to be treated either fusion proteins with a pro-inflammatory or an anti-inflammatory cytokine may be favored. For example, for the treatment of inflammatory diseases in general fusion constructs comprising anti-inflammatory cytokines are preferred, whereas for the treatment of cancer in general fusion constructs comprising pro-inflammatory cytokines are preferred.

The chemokine is preferably selected from the group consisting of IL-8, GRO alpha, GRO beta, GRO gamma, ENA-78, LDGF-PBP, GCP-2, PF4, Mig, IP-10, SDF-1alpha/beta, BUNZO/STRC33, I-TAC, BLC/BCA-1, MIP-1alpha, MIP-1 beta, MDC, TECK, TARC, RANTES, HCC-1, HCC-4, DC-CK1, MIP-3 alpha, MIP-3 beta, MCP-1-5, eotaxin, Eotaxin-2, I-309, MPIF-1, 6Ckine, CTACK, MEC, lymphotactin and fractalkine. The major role of chemokines is to act as a chemoattractant to guide the migration of cells. Cells that are attracted by chemokines follow a signal of increasing chemokine concentration towards the source of the chemokine. It follows that within the fusion protein the chemokin can be used to guide the migration of the protein or peptide of the invention, e.g. to a specific cells type or body site.

The pro-coagulant factor is preferably a tissue factor. A pro-coagulant factor promoting the process by which blood changes from a liquid to a gel, forming a blood clot. Pro-coagulant factors may, for example, aid in wound healing.

The proteinaceous toxic compound is preferably Ricin-A chain, modeccin, truncated Pseudomonas exotoxin A, diphtheria toxin and recombinant gelonin. Toxic compounds can have a toxic effect on a whole organism as well as on a substructure of the organism, such as a particular cell type. Toxic compounds are frequently used in the treatment of tumors. Tumor cells generally grow faster than normal body cells, so that they preferentially accumulate toxic compounds and in higher amounts.

The enzyme for pro-drug activation is preferably an enzyme selected from the group consisting of carboxy-peptidases, glucuronidases and glucosidases. Among the broad array of genes that have been evaluated for tumor therapy, those encoding pro-drug activation enzymes are especially appealing as they directly complement ongoing clinical chemotherapeutic regimes. These enzymes can activate prodrugs that have low inherent toxicity using both bacterial and yeast enzymes, or enhance prodrug activation by mammalian enzymes.

In accordance with a preferred embodiment, the protein or peptide is fused to a heterologous non-proteinaceous compound.

As used herein, a heterologous compound is a compound that cannot be found in nature fused to the amino acid sequence of SEQ ID NO: 1.

The heterologous non-proteinaceous compound can either be directly or indirectly fused to the nucleic acid molecule of the invention. For, example chemical linker may be used. Chemical linkers may contain diverse functional groups, such as primary amines, sulfhydryls, acids, alcohols and bromides. Many of our crosslinkers are functionalized with maleimide (sulfhydral reactive) and succinimidyl ester (NHS) or isothiocyanate (ITC) groups that react with amines.

The heterologous non-proteinaceous compound is preferably a pharmaceutically active compound or diagnostically active compound. The pharmaceutically active compound or diagnostically active compound is preferably selected from the group consisting of (a) a fluorescent dye, (b) a photosensitizer, (c) a radionuclide, (d) a contrast agent for medical imaging, (e) a toxic compound, or (f) an ACE inhibitor, a Renin inhibitor, an ADH inhibitor, an Aldosteron inhibitor, an Angiotensin receptor blocker, a TSH-receptor, a LH-/HCG-receptor, an oestrogen receptor, a progesterone receptor, an androgen receptor, a GnRH-receptor, a GH (growth hormone) receptor, or a receptor for IGF-I or IGF-II.

The fluorescent dye is preferably a component selected from Alexa Fluor or Cy dyes.

The photosensitizer is preferably phototoxic red fluorescent protein KillerRed or haematoporphyrin.

The radionuclide is preferably either selected from the group of gamma-emitting isotopes, more preferably ^{99m}Tc, ¹²³I, ¹¹¹In, and/or from the group of positron emitters, more preferably ¹⁸F, ⁶⁴Cu, ⁶⁸Ga, ⁸⁶Y, ¹²⁴I, and/or from the group of beta-emitter, more preferably ¹³¹I, ⁹⁰Y, ¹⁷⁷ Lu, ⁶⁷Cu, ⁹⁰Sr, or from the group of alpha-emitter, preferably ²¹³Bi, ²¹¹At.

A contrast agent as used herein is a substance used to enhance the contrast of structures or fluids within the body in medical imaging. Common contrast agents work based on X-ray attenuation and magnetic resonance signal enhancement.

The toxic compound is preferably a small organic compound, more preferably a toxic compound selected from the group consisting of calicheamicin, maytansinoid, neocarzinostatin, esperamicin, dynemicin, kedarcidin, maduropeptin, doxorubicin, daunorubicin, and auristatin. In contrast to the herein above-described proteinaceous toxic compound these toxic compounds are non-proteinaceous.

In a second aspect the invention relates to an inhibitor of the nucleic acid molecule of the the first aspect and/or a binding molecule, preferably an inhibitor of the protein as defined in the first aspect for use in the treatment of a tumor, wherein (i) the inhibitor of the nucleic acid molecule as defined in the first aspect of the invention is selected from an aptamer, a siRNA, a shRNA, a miRNA, a ribozyme, an antisense nucleic acid molecule, a CRISPR-Cas9-based construct, a CRISPR-Cpf1-based construct, a meganuclease, a zinc finger nuclease, and a transcription activator-like (TAL) effector (TALE) nuclease, and/or (ii) the binding molecule, preferably the inhibitor of the protein as defoned in the first aspect of the invention is selected from an antibody or antibody mimetic, and an aptamer, wherein the antibody mimetic is preferably selected from affibodies, adnectins, anticalins, DARPins, avimers, nanofitins, affilins, Kunitz domain peptides, Fynomers^{®}, trispecific binding molecules and probodies.

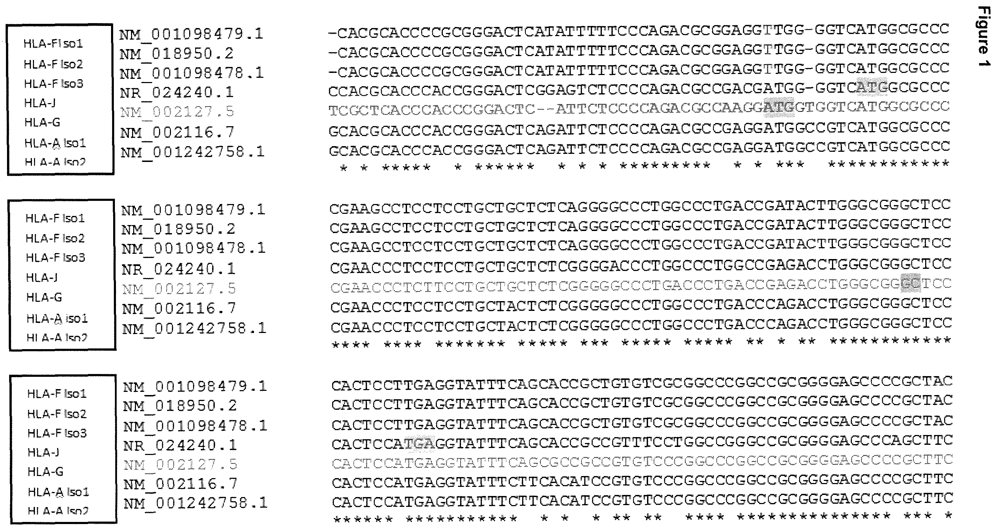

A binding molecule of the protein as defined in the first aspect is a compound being capable of binding to the protein as defined in the first aspect. The binding molecule preferably specifically binds to the protein as defined in the first aspect. Specific binding designates that the binding molecule essentially does not or essentially does not bind to other proteins or peptides than the protein as defined in the first aspect. In particular, it is preferred that the binding molecule is not capable to bind to other HLA proteins than HLA-J. The binding molecule preferably binds to the C-terminal part of HLA-J, i.e. C-terminally of the wrongly assumed stop as reported in Messer at al., J Immunol. 1992 Jun 15; 148(12):4043-53. The exatct position of the wrongly assumed stop is shown Figure 1 and further discussed in Example 1. A binding molecule of the protein as defined in the first aspect is, for example, suitable for research purposes. For example, an antibody binding to the protein as defined in the first aspect can be used in immuonassays, such as an ELISA or Western Blot. This is illustrated by Example 4. Immunoassays are biochemical tests that can measure the presence or concentration the protein as defined in the first aspect in a sample (e.g. a solution). The binding molecule of the protein as defined in the first aspect is preferably capable of inhibiting the protein as defined in the first aspect. In this case the binding molecule is designated inhibitor.

A compound inhibiting the expression of the nucleic acid molecule and/or the protein of the invention is in accordance with the present invention (i) a compound lowering or preventing the transcription of the gene encoding the nucleic acid molecule and/or the protein of the invention, or (ii) is acompound lowering or preventing the translation of the mRNA encoding the protein of the invention. Compounds of (i) include compounds interfering with the transcriptional machinery and/or its interaction with the promoter of said gene and/or with expression control elements remote from the promoter such as enhancers. Compounds of (ii) include compounds interfering with the translational machinery. The compound inhibiting the expression of the nucleic acid molecule and/or the protein of the invention specifically inhibits the expression of the nucleic acid molecule and/or the protein of the invention, for example, by specifically interfering with the promoter region controlling the expression. Preferably, the transcription of the nucleic acid molecule and/or the protein of the invention or the translation of the protein of the invention is reduced by at least 50%, more preferred at least 75% such as at least 90% or 95%, even more preferred at least 98% and most preferred by about 100% (e.g., as compared to the same experimental set up in the absence of the compound).

A compound inhibiting the activity of the nucleic acid molecule and/or the protein of the invention in accordance with the present invention causes said nucleic acid molecule and/or protein to perform its/their function with lowered efficiency. The compound inhibiting the activity of the nucleic acid molecule and/or the protein of the invention specifically inhibits the activity of said nucleic acid molecule and/or protein. As will be further detailed herein below, the compound inhibiting the activity of the nucleic acid molecule and/or the protein of the invention may specifically inhibit the activity of said nucleic acid molecule and/or protein by interacting with the nucleic acid molecule and/or protein itself or by specifically inhibiting (preferably killing) cells that produce said nucleic acid molecule and/or produce said protein and/or bind to said protein. Preferably, the activity of the nucleic acid molecule and/or the protein of the invention is reduced by at least 50%, more preferred at least 75% such as at least 90% or 95%, even more preferred at least 98%, and most preferably about 100% (e.g., as compared to the same experimental set up in the absence of the compound).

The activity of the nucleic acid molecule and/or the protein of the invention is in accordance with this invention, preferably its/their capability to induce resistance to chemotherapy in cancer patients and/or to reduce progression free as well as overall survival in cancer patients (see also the appended examples). The chemotherapy as referred to herein may be an adjuvant chemotherapy or a neoadjuvant chemotherapy, and is preferably a neoadjuvant chemotherapy. Chemotherapy uses drugs to destroy cancer cells, stop their growth, or ameliorate symptoms. In neoadjuvant (also called preoperative or primary) chemotherapy, drug treatment takes place before surgical extraction of a tumor. This is in contrast with adjuvant chemotherapy, which is drug treatment after surgery. Means and methods for determining this activity are established in the art and are illustrated in the examples herein below. In accordance with the medical aspects of the invention, these activities of the nucleic acid molecule and/or the protein of the invention are therefore to be inhibited.

The efficiency of inhibition of an inhibitor can be quantified by methods comparing the level of activity in the presence of the inhibitor to that in the absence of the inhibitor. For example, the change in the amount of the nucleic acid molecule and/or the protein of the invention formed may be used in the measurement. The efficiency of several inhibitors may be determined simultaneously in high-throughput formats. High-throughput assays, independently of being biochemical, cellular or other assays, generally may be performed in wells of microtiter plates, wherein each plate may contain 96, 384 or 1536 wells. Handling of the plates, including incubation at temperatures other than ambient temperature, and bringing into contact of test compounds with the assay mixture is preferably effected by one or more computer-controlled robotic systems including pipetting devices. In case large libraries of test compounds are to be screened and/or screening is to be effected within a short time, mixtures of, for example 10, 20, 30, 40, 50 or 100 test compounds may be added to each well. In case a well exhibits the expected activity, said mixture of test compounds may be de-convoluted to identify the one or more test compounds in said mixture giving rise to said activity.

The compounds inhibiting the expression and/or the activity of the nucleic acid molecule and/or the protein of the invention may be formulated as vesicles, such as liposomes or exososmes. Liposomes have attracted great interest because of their specificity and the duration of action they offer from the standpoint of drug delivery. Liposomal cell-type delivery systems have been used to effectively deliver nucleic acids, such as siRNA in vivo into cells (Zimmermann et al. (2006) Nature, 441:111-114). Liposomes are unilamellar or multilamellar vesicles which have a membrane formed from a lipophilic material and an aqueous interior. The aqueous portion contains the composition to be delivered. Cationic liposomes possess the advantage of being able to fuse to the cell wall. Non-cationic liposomes, although not able to fuse as efficiently with the cell wall, are phagocytosed by macrophages and other cells in vivo. Exosomes are lipid packages which can carry a variety of different molecules including RNA (Alexander et al. (2015), Nat Commun; 6:7321). The exosomes including the molecules comprised therein can be taken up by recipient cells. Hence, exosomes are important mediators of intercellular communication and regulators of the cellular niche. Exosomes are useful for diagnostic and therapeutic purposes, since they can be used as delivery vehicles, e.g. for contrast agents or drugs.

The compounds inhibiting the expression and/or the activity of the nucleic acid molecule and/or the protein of the invention can be administered to the subject at a suitable dose and/or a therapeutically effective amount. This will be further discussed herein below in connection with the pharmaceutical composition of the invention.

The length of treatment needed to observe changes and the interval following treatment for responses to occur vary depending on the desired effect. The particular amounts may be determined by conventional tests which are well known to the person skilled in the art. Suitable tests are, for example, described in Tamhane and Logan (2002), "Multiple Test Procedures for Identifying the Minimum Effective and Maximum Safe Doses of a Drug", Journal of the American statistical association, 97(457):1-9.

The compounds inhibiting the expression and/or the activity of the nucleic acid molecule and/or the protein of the invention are preferably admixed with a pharmaceutically acceptable carrier or excipient to form a pharmaceutical composition. Suitable pharmaceutically acceptable carriers or excipients as well as the formulation of pharmaceutical compositions will be discussed herein below in connection with the pharmaceutical composition of the invention.

The "small molecule" as used herein is preferably an organic molecule. Organic molecules relate or belong to the class of chemical compounds having a carbon basis, the carbon atoms linked together by carbon-carbon bonds. The original definition of the term organic related to the source of chemical compounds, with organic compounds being those carbon-containing compounds obtained from plant or animal or microbial sources, whereas inorganic compounds were obtained from mineral sources. Organic compounds can be natural or synthetic. The organic molecule is preferably an aromatic molecule and more preferably a heteroaromatic molecule. In organic chemistry, the term aromaticity is used to describe a cyclic (ring-shaped), planar (flat) molecule with a ring of resonance bonds that exhibits more stability than other geometric or connective arrangements with the same set of atoms. Aromatic molecules are very stable, and do not break apart easily to react with other substances. In a heteroaromatic molecule at least one of the atoms in the aromatic ring is an atom other than carbon, e.g. N, S, or O. For all above-described organic molecules the molecular weight is preferably in the range of 200 Da to 1500 Da and more preferably in the range of 300 Da to 1000 Da.

Alternatively, the "small molecule" in accordance with the present invention may be an inorganic compound. Inorganic compounds are derived from mineral sources and include all compounds without carbon atoms (except carbon dioxide, carbon monoxide and carbonates). Preferably, the small molecule has a molecular weight of less than about 2000 Da, or less than about 1000 Da such as less than about 500 Da, and even more preferably less than about Da amu. The size of a small molecule can be determined by methods well-known in the art, e.g., mass spectrometry. The small molecules may be designed, for example, based on the crystal structure of the target molecule, where sites presumably responsible for the biological activity can be identified and verified in in vivo assays such as in vivo high-throughput screening (HTS) assays.

The term "antibody" as used in accordance with the present invention comprises, for example, polyclonal or monoclonal antibodies. Furthermore, also derivatives or fragments thereof, which still retain the binding specificity to the target, e.g. the HLA-J protein of SEQ ID NO: 1, are comprised in the term "antibody". Antibody fragments or derivatives comprise, inter alia, Fab or Fab' fragments, Fd, F(ab')2, Fv or scFv fragments, single domain VH or V-like domains, such as VhH or V-NAR-domains, as well as multimeric formats such as minibodies, diabodies, tribodies or triplebodies, tetrabodies or chemically conjugated Fab'-multimers (see, for example, Harlow and Lane "Antibodies, A Laboratory Manual", Cold Spring Harbor Laboratory Press, 198; Harlow and Lane "Using Antibodies: A Laboratory Manual" Cold Spring Harbor Laboratory Press, 1999; Altshuler EP, Serebryanaya DV, Katrukha AG. 2010, Biochemistry (Mosc)., vol. 75(13), 1584; Holliger P, Hudson PJ. 2005, Nat Biotechnol., vol. 23(9), 1126). The multimeric formats in particular comprise bispecific antibodies that can simultaneously bind to two different types of antigen. The first antigen can be found on the protein of the invention. The second antigen may, for example, be a tumor marker that is specifically expressed on cancer cells or a certain type of cancer cells. Non-limting examples of bispecific antibodies formats are Biclonics (bispecific, full length human IgG antibodies), DART (Dual-affinity Re-targeting Antibody) and BiTE (consisting of two single-chain variable fragments (scFvs) of different antibodies) molecules (Kontermann and Brinkmann (2015), Drug Discovery Today, 20(7):838-847).

The term "antibody" also includes embodiments such as chimeric (human constant domain, non-human variable domain), single chain and humanised (human antibody with the exception of non-human CDRs) antibodies.

Various techniques for the production of antibodies are well known in the art and described, e.g. in Harlow and Lane (1988) and (1999) and Altshuler et al., 2010, loc. cit. Thus, polyclonal antibodies can be obtained from the blood of an animal following immunisation with an antigen in mixture with additives and adjuvants and monoclonal antibodies can be produced by any technique which provides antibodies produced by continuous cell line cultures. Examples for such techniques are described, e.g. in Harlow E and Lane D, Cold Spring Harbor Laboratory Press, 1988; Harlow E and Lane D, Using Antibodies: A Laboratory Manual, Cold Spring Harbor Laboratory Press, 1999 and include the hybridoma technique originally described by Köhler and Milstein, 1975, the trioma technique, the human B-cell hybridoma technique (see e.g. Kozbor D, 1983, Immunology Today, vol.4, 7; Li J, et al. 2006, PNAS, vol. 103(10), 3557) and the EBV-hybridoma technique to produce human monoclonal antibodies (Cole et al., 1985, Alan R. Liss, Inc, 77-96). Furthermore, recombinant antibodies may be obtained from monoclonal antibodies or can be prepared de novo using various display methods such as phage, ribosomal, mRNA, or cell display. A suitable system for the expression of the recombinant (humanised) antibodies may be selected from, for example, bacteria, yeast, insects, mammalian cell lines or transgenic animals or plants (see, e.g., US patent 6,080,560; Holliger P, Hudson PJ. 2005, Nat Biotechnol., vol. 23(9), 11265). Further, techniques described for the production of single chain antibodies (see, inter alia, US Patent 4,946,778) can be adapted to produce single chain antibodies specific for an epitope of HLA-J. Surface plasmon resonance as employed in the BIAcore system can be used to increase the efficiency of phage antibodies.

As used herein, the term "antibody mimetics" refers to compounds which, like antibodies, can specifically bind antigens, such the HLA-J protein of SEQ ID NO: 1 in the present case, but which are not structurally related to antibodies. Antibody mimetics are usually artificial peptides or proteins with a molar mass of about 3 to 20 kDa. For example, an antibody mimetic may be selected from the group consisting of affibodies, adnectins, anticalins, DARPins, avimers, nanofitins, affilins, Kunitz domain peptides, Fynomers^{®}, trispecific binding molecules and prododies. These polypeptides are well known in the art and are described in further detail herein below.

The term "affibody", as used herein, refers to a family of antibody mimetics which is derived from the Z-domain of staphylococcal protein A. Structurally, affibody molecules are based on a three-helix bundle domain which can also be incorporated into fusion proteins. In itself, an affibody has a molecular mass of around 6kDa and is stable at high temperatures and under acidic or alkaline conditions. Target specificity is obtained by randomisation of 13 amino acids located in two alpha-helices involved in the binding activity of the parent protein domain (Feldwisch J, Tolmachev V.; (2012) Methods Mol Biol. 899:103-26).

The term "adnectin" (also referred to as "monobody"), as used herein, relates to a molecule based on the 10th extracellular domain of human fibronectin III (10Fn3), which adopts an Ig-like β-sandwich fold of 94 residues with 2 to 3 exposed loops, but lacks the central disulphide bridge (Gebauer and Skerra (2009) Curr Opinion in Chemical Biology 13:245-255). Adnectins with the desired target specificity, i.e. against HLA-J, can be genetically engineered by introducing modifications in specific loops of the protein.

The term "anticalin", as used herein, refers to an engineered protein derived from a lipocalin (Beste G, Schmidt FS, Stibora T, Skerra A. (1999) Proc Natl Acad Sci U S A. 96(5):1898-903; Gebauer and Skerra (2009) Curr Opinion in Chemical Biology 13:245-255). Anticalins possess an eight-stranded β-barrel which forms a highly conserved core unit among the lipocalins and naturally forms binding sites for ligands by means of four structurally variable loops at the open end. Anticalins, although not homologous to the IgG superfamily, show features that so far have been considered typical for the binding sites of antibodies: (i) high structural plasticity as a consequence of sequence variation and (ii) elevated conformational flexibility, allowing induced fit to targets with differing shape.

As used herein, the term "DARPin" refers to a designed ankyrin repeat domain (166 residues), which provides a rigid interface arising from typically three repeated β-turns. DARPins usually carry three repeats corresponding to an artificial consensus sequence, wherein six positions per repeat are randomised. Consequently, DARPins lack structural flexibility (Gebauer and Skerra, 2009).

The term "avimer", as used herein, refers to a class of antibody mimetics which consist of two or more peptide sequences of 30 to 35 amino acids each, which are derived from A-domains of various membrane receptors and which are connected by linker peptides. Binding of target molecules occurs via the A-domain and domains with the desired binding specificity, i.e. for HLA-J, can be selected, for example, by phage display techniques. The binding specificity of the different A-domains contained in an avimer may, but does not have to be identical (Weidle UH, et al., (2013), Cancer Genomics Proteomics; 10(4):155-68).

A "nanofitin" (also known as affitin) is an antibody mimetic protein that is derived from the DNA binding protein Sac7d of Sulfolobus acidocaldarius. Nanofitins usually have a molecular weight of around 7kDa and are designed to specifically bind a target molecule, such as e.g. HLA-J, by randomising the amino acids on the binding surface (Mouratou B, Béhar G, Paillard-Laurance L, Colinet S, Pecorari F., (2012) Methods Mol Biol.; 805:315-31).

The term "affilin", as used herein, refers to antibody mimetics that are developed by using either gamma-B crystalline or ubiquitin as a scaffold and modifying amino-acids on the surface of these proteins by random mutagenesis. Selection of affilins with the desired target specificity, i.e. against HLA-J, is effected, for example, by phage display or ribosome display techniques. Depending on the scaffold, affilins have a molecular weight of approximately 10 or 20kDa. As used herein, the term affilin also refers to di- or multimerised forms of affilins (Weidle UH, et al., (2013), Cancer Genomics Proteomics; 10(4):155-68).

A "Kunitz domain peptide" is derived from the Kunitz domain of a Kunitz-type protease inhibitor such as bovine pancreatic trypsin inhibitor (BPTI), amyloid precursor protein (APP) or tissue factor pathway inhibitor (TFPI). Kunitz domains have a molecular weight of approximately 6kDA and domains with the required target specificity, i.e. against HLA-J, can be selected by display techniques such as phage display (Weidle et al., (2013), Cancer Genomics Proteomics; 10(4):155-68).

As used herein, the term "Fynomer^{®}" refers to a non-immunoglobulin-derived binding polypeptide derived from the human Fyn SH3 domain. Fyn SH3-derived polypeptides are well-known in the art and have been described e.g. in Grabulovski et al. (2007) JBC, 282, p. 3196-3204, WO 2008/022759, Bertschinger et al (2007) Protein Eng Des Sel 20(2):57-68, Gebauer and Skerra (2009) Curr Opinion in Chemical Biology 13:245-255, or Schlatter et al. (2012), MAbs 4:4, 1-12).

The term "trispecific binding molecule" as used herein refers to a polypeptide molecule that possesses three binding domains and is thus capable of binding, preferably specifically binding to three different epitopes. At least one of these three epitopes is an epitope of the protein as defined in the first aspectof the invention. The two other epitopes may also be epitopes of the protein as defined in the first aspectof the invention or may be epitopes of one or two different antigens. The trispecific binding molecule is preferably a TriTac. A TriTac is a T-cell engager for solid tumors which comprised of three binding domains being designed to have an extended serum half-life and be about one-third the size of a monoclonal antibody.

As used herein, the term "probody" refers to a protease-activatable antibody prodrug. A probody consists of an authentic IgG heavy chain and a modified light chain. A masking peptide is fused to the light chain through a peptide linker that is cleavable by tumor-specific proteases. The masking peptide prevents the probody binding to healthy tissues, thereby minimizing toxic side effects.

Aptamers are nucleic acid molecules or peptide molecules that bind a specific target molecule. Aptamers are usually created by selecting them from a large random sequence pool, but natural aptamers also exist in riboswitches. Aptamers can be used for both basic research and clinical purposes as macromolecular drugs. Aptamers can be combined with ribozymes to self-cleave in the presence of their target molecule. These compound molecules have additional research, industrial and clinical applications (Osborne et. al. (1997), Current Opinion in Chemical Biology, 1:5-9; Stull & Szoka (1995), Pharmaceutical Research, 12, 4:465-483).

Nucleic acid aptamers are nucleic acid species that normally consist of (usually short) strands of oligonucleotides. Typically, they have been engineered through repeated rounds of in vitro selection or equivalently, SELEX (systematic evolution of ligands by exponential enrichment) to bind to various molecular targets such as small molecules, proteins, nucleic acids, and even cells, tissues and organisms.

Peptide aptamers are usually peptides or proteins that are designed to interfere with other protein interactions inside cells. They consist of a variable peptide loop attached at both ends to a protein scaffold. This double structural constraint greatly increases the binding affinity of the peptide aptamer to levels comparable to an antibody's (nanomolar range). The variable peptide loop typically comprises 10 to 20 amino acids, and the scaffold may be any protein having good solubility properties. Currently, the bacterial protein Thioredoxin-A is the most commonly used scaffold protein, the variable peptide loop being inserted within the redox-active site, which is a -Cys-Gly-Pro-Cys-loop (SEQ ID NO: 55) in the wild protein, the two cysteins lateral chains being able to form a disulfide bridge. Peptide aptamer selection can be made using different systems, but the most widely used is currently the yeast two-hybrid system.

Aptamers offer the utility for biotechnological and therapeutic applications as they offer molecular recognition properties that rival those of the commonly used biomolecules, in particular antibodies. In addition to their discriminatory recognition, aptamers offer advantages over antibodies as they can be engineered completely in a test tube, are readily produced by chemical synthesis, possess desirable storage properties, and elicit little or no immunogenicity in therapeutic applications. Non-modified aptamers are cleared rapidly from the bloodstream, with a half-life of minutes to hours, mainly due to nuclease degradation and clearance from the body by the kidneys, a result of the aptamers' inherently low molecular weight. Unmodified aptamer applications currently focus on treating transient conditions such as blood clotting, or treating organs such as the eye where local delivery is possible. This rapid clearance can be an advantage in applications such as in vivo diagnostic imaging. Several modifications, such as 2'-fluorine-substituted pyrimidines, polyethylene glycol (PEG) linkage, fusion to albumin or other half life extending proteins etc. are available to scientists such that the half-life of aptamers can be increased for several days or even weeks.

As discussed, the above-described small molecule, antibody or antibody mimetic and aptamer can specifically bind to the protein as defined in the first aspect of the invention. This binding may block the immunosuppressive properties of the protein as defined in the first aspect of the invention and preferably its capability to induce resistance to chemotherapy in cancer patients and/or to reduce progression free as well as overall survival in cancer patients. In this case the small molecule, antibody or antibody mimetic and aptamer are also referred to as blocking small molecule, antibody or antibody mimetic and aptamer. A blocking small molecule, antibody or antibody mimetic and aptamer blocks interactions of the protein as defined in the first aspect of the invention with other cellular components, such as ligands and receptor which normally interact with the protein as defined in the first aspect of the invention.

The small molecule, antibody or antibody mimetic and aptamer can also be generated in the format of drug-conjugates. In this case the small molecule, antibody or antibody mimetic and aptamer in itself may not have an inhibitory effect but the inhibitory effect is only conferred by the drug. The small molecule, antibody or antibody mimetic and aptamer confer the site-specificity binding of the drug to cells producing and/or binding to the protein of the invention. The drug is preferably capable to kill cells producing and/or binding to the protein of the invention. Hence, by combining the targeting capabilities of moelcules binding to the protein of the invention with the cell-killing ability of the drug, the drug conjugates become inhibors that allow for discrimination between healthy and diseased tissue and cells. Cleavable and non-cleavable linkers to design drug conjugates are known in the art. Non-limiting examples of drugs being capable of killing cells are cytostatic drugs and radioisotopes that deliver radiation directly to the cancer cells.

It is furthermore possible to confine the binding and/or inhibitory activity of the small molecule, antibody or antibody mimetic and aptamer to certain tissues or cell-types, in particular diseased tissues or cell-types. For instance, probodies may be designed. In a probody the small molecule, antibody or antibody mimetic or aptamer is bound to a masking peptide which limits or prevents binding to the protein of the invention and which masking peptide can be cleaved by a protease. Proteases are enzymes that digest proteins into smaller pieces by cleaving specific amino acid sequences known as substrates. In normal healthy tissue, protease activity is tightly controlled. In cancer cells, protease activity is upregulated. In healthy tissue or cells, where protease activity is regulated and minimal, the target-binding region of the probody remains masked and is thus unable to bind. On the other hand, in diseased tissue or cells, where protease activity is upregulated, the target-binding region of the probody gets unmasked and is thus able to bind and/or inhibit.

In accordance with the present invention, the term "small interfering RNA (siRNA)", also known as short interfering RNA or silencing RNA, refers to a class of 18 to 30, preferably 19 to 25, most preferred 21 to 23 or even more preferably 21 nucleotide-long double-stranded RNA molecules that play a variety of roles in biology. Most notably, siRNA is involved in the RNA interference (RNAi) pathway where the siRNA interferes with the expression of a specific gene. In addition to their role in the RNAi pathway, siRNAs also act in RNAi-related pathways, e.g. as an antiviral mechanism or in shaping the chromatin structure of a genome.

siRNAs naturally found in nature have a well defined structure: a short double-strand of RNA (dsRNA) with 2-nt 3' overhangs on either end. Each strand has a 5' phosphate group and a 3' hydroxyl (-OH) group. This structure is the result of processing by dicer, an enzyme that converts either long dsRNAs or small hairpin RNAs into siRNAs. siRNAs can also be exogenously (artificially) introduced into cells to bring about the specific knockdown of a gene of interest. Essentially any gene for which the sequence is known can thus be targeted based on sequence complementarity with an appropriately tailored siRNA. The double-stranded RNA molecule or a metabolic processing product thereof is capable of mediating target-specific nucleic acid modifications, particularly RNA interference and/or DNA methylation. Exogenously introduced siRNAs may be devoid of overhangs at their 3' and 5' ends, however, it is preferred that at least one RNA strand has a 5'- and/or 3'-overhang. Preferably, one end of the double-strand has a 3'-overhang from 1 to 5 nucleotides, more preferably from 1 to 3 nucleotides and most preferably 2 nucleotides. The other end may be blunt-ended or has up to 6 nucleotides 3'-overhang. In general, any RNA molecule suitable to act as siRNA is envisioned in the present invention. The most efficient silencing was so far obtained with siRNA duplexes composed of 21-nt sense and 21-nt antisense strands, paired in a manner to have a 2-nt 3'- overhang. The sequence of the 2-nt 3' overhang makes a small contribution to the specificity of target recognition restricted to the unpaired nucleotide adjacent to the first base pair (Elbashir et al. 2001). 2'-deoxynucleotides in the 3' overhangs are as efficient as ribonucleotides, but are often cheaper to synthesize and probably more nuclease resistant. Delivery of siRNA may be accomplished using any of the methods known in the art, for example by combining the siRNA with saline and administering the combination intravenously or intranasally or by formulating siRNA in glucose (such as for example 5% glucose) or cationic lipids and polymers can be used for siRNA delivery *in vivo* through systemic routes either intravenously (IV) or intraperitoneally (IP) (Fougerolles et al. (2008), Current Opinion in Pharmacology, 8:280-285; Lu et al. (2008), Methods in Molecular Biology, vol. 437: Drug Delivery Systems - Chapter 3: Delivering Small Interfering RNA for Novel Therapeutics).

A short hairpin RNA (shRNA) is a sequence of RNA that makes a tight hairpin turn that can be used to silence gene expression via RNA interference. shRNA uses a vector introduced into cells and utilizes the U6 promoter to ensure that the shRNA is always expressed. This vector is usually passed on to daughter cells, allowing the gene silencing to be inherited. The shRNA hairpin structure is cleaved by the cellular machinery into siRNA, which is then bound to the RNA-induced silencing complex (RISC). This complex binds to and cleaves mRNAs which match the siRNA that is bound to it. si/shRNAs to be used in the present invention are preferably chemically synthesized using appropriately protected ribonucleoside phosphoramidites and a conventional DNA/RNA synthesizer. Suppliers of RNA synthesis reagents are Proligo (Hamburg, Germany), Dharmacon Research (Lafayette, CO, USA), Pierce Chemical (part of Perbio Science, Rockford, IL, USA), Glen Research (Sterling, VA, USA), ChemGenes (Ashland, MA, USA), and Cruachem (Glasgow, UK). Most conveniently, siRNAs or shRNAs are obtained from commercial RNA oligo synthesis suppliers, which sell RNA-synthesis products of different quality and costs. In general, the RNAs applicable in the present invention are conventionally synthesized and are readily provided in a quality suitable for RNAi.

Further molecules effecting RNAi include, for example, microRNAs (miRNA). Said RNA species are single-stranded RNA molecules. Endogenously present miRNA molecules regulate gene expression by binding to a complementary mRNA transcript and triggering of the degradation of said mRNA transcript through a process similar to RNA interference. Accordingly, exogenous miRNA may be employed as an inhibitor of HLA-J after introduction into the respective cells.

A ribozyme (from ribonucleic acid enzyme, also called RNA enzyme or catalytic RNA) is an RNA molecule that catalyses a chemical reaction. Many natural ribozymes catalyse either their own cleavage or the cleavage of other RNAs, but they have also been found to catalyse the aminotransferase activity of the ribosome. Non-limiting examples of well-characterised small self-cleaving RNAs are the hammerhead, hairpin, hepatitis delta virus, and *in vitro-*selected lead-dependent ribozymes, whereas the group I intron is an example for larger ribozymes. The principle of catalytic self-cleavage has become well established in recent years. The hammerhead ribozymes are characterised best among the RNA molecules with ribozyme activity. Since it was shown that hammerhead structures can be integrated into heterologous RNA sequences and that ribozyme activity can thereby be transferred to these molecules, it appears that catalytic antisense sequences for almost any target sequence can be created, provided the target sequence contains a potential matching cleavage site. The basic principle of constructing hammerhead ribozymes is as follows: A region of interest of the RNA, which contains the GUC (or CUC) triplet, is selected. Two oligonucleotide strands, each usually with 6 to 8 nucleotides, are taken and the catalytic hammerhead sequence is inserted between them. The best results are usually obtained with short ribozymes and target sequences.

A recent development, also useful in accordance with the present invention, is the combination of an aptamer, recognizing a small compound, with a hammerhead ribozyme. The conformational change induced in the aptamer upon binding the target molecule can regulate the catalytic function of the ribozyme.

The term "antisense nucleic acid molecule", as used herein, refers to a nucleic acid which is complementary to a target nucleic acid. An antisense molecule in accordance with the invention is capable of interacting with the target nucleic acid, more specifically it is capable of hybridizing with the target nucleic acid. Due to the formation of the hybrid, transcription of the target gene(s) and/or translation of the target mRNA is reduced or blocked. Standard methods relating to antisense technology have been described (see, e.g., Melani et al., Cancer Res. (1991) 51:2897-2901).

CRISPR/Cas9, as well as CRISPR-Cpf1, technologies are applicable in nearly all cells/model organisms and can be used for knock out mutations, chromosomal deletions, editing of DNA sequences and regulation of gene expression. The regulation of the gene expression can be manipulated by the use of a catalytically dead Cas9 enzyme (dCas9) that is conjugated with a transcriptional repressor to repress transcription a specific gene, here the HLA-J gene. Similarly, catalytically inactive, "dead" Cpf1 nuclease (CRISPR from Prevotella and Francisella-1) can be fused to synthetic transcriptional repressors or activators to downregulate endogenous promoters, e.g. the promoter which controls HLA-J expression. Alternatively, the DNA-binding domain of zinc finger nucleases (ZFNs) or transcription activator-like effector nucleases (TALENs) can be designed to specifically recognize the HLA-J gene or its promoter region or its 5'-UTR thereby inhibiting the expression of the HLA-J gene.

Inhibitors provided as inhibiting nucleic acid molecules that target the HLA-J gene or a regulatory molecule involved in HLA-J expression are also envisaged herein. Such molecules, which reduce or abolish the expression of HLA-J or a regulatory molecule include, without being limiting, meganucleases, zinc finger nucleases and transcription activator-like (TAL) effector (TALE) nucleases. Such methods are described in Silva et al., Curr Gene Ther. 2011;11(1):11-27; Miller et al., Nature biotechnology. 2011;29(2):143-148, and Klug, Annual review of biochemistry. 2010; 79:213-231.

Also described herein is a composition, preferably a pharmaceutical or diagnostic composition, comprising the nucleic acid molecule, the vector, the host cell, the protein or peptide, the binding molecule, preferably the inhibitor of the invention or combinations thereof.

The term "composition" as used herein refers to a composition comprising at least one of the nucleic acid molecule, the vector, the host cell, the protein or peptide of, the binding molecule, preferably the inhibitor of the second aspect invention, or combinations thereof which are also collectively referred in the following as compounds.

In accordance with the present invention, the term "pharmaceutical composition" relates to a composition for administration to a patient, preferably a human patient. The pharmaceutical composition of the invention comprises the compounds recited above. It may, optionally, comprise further molecules capable of altering the characteristics of the compounds of the invention thereby, for example, stabilizing, modulating and/or activating their function. The composition may be in solid, liquid or gaseous form and may be, inter alia, in the form of (a) powder(s), (a) tablet(s), (a) solution(s) or (an) aerosol(s). The pharmaceutical composition of the present invention may, optionally and additionally, comprise a pharmaceutically acceptable carrier. Examples of suitable pharmaceutical carriers are well known in the art and include phosphate buffered saline solutions, water, emulsions, such as oil/water emulsions, various types of wetting agents, sterile solutions, organic solvents including DMSO etc. Compositions comprising such carriers can be formulated by well known conventional methods. These pharmaceutical compositions can be administered to the subject at a suitable dose. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. The therapeutically effective amount for a given situation will readily be determined by routine experimentation and is within the skills and judgement of the ordinary clinician or physician. Generally, the regimen as a regular administration of the pharmaceutical composition should be in the range of 1 µg to 5 g units per day. However, a more preferred dosage might be in the range of 0.01 mg to 100 mg, even more preferably 0.01 mg to 50 mg and most preferably 0.01 mg to 10 mg per day. Furthermore, if for example said compound is an iRNA agent, such as an siRNA, the total pharmaceutically effective amount of pharmaceutical composition administered will typically be less than about 75 mg per kg of body weight, such as for example less than about 70, 60, 50, 40, 30, 20, 10, 5, 2, 1, 0.5, 0.1, 0.05, 0.01, 0.005, 0.001, or 0.0005 mg per kg of body weight. More preferably, the amount will be less than 2000 nmol of iRNA agent (e.g., about 4.4 x 10¹⁶ copies) per kg of body weight, such as for example less than 1500, 750, 300, 150, 75, 15, 7.5, 1.5, 0.75, 0.15, 0.075, 0.015, 0.0075, 0.0015, 0.00075 or 0.00015 nmol of iRNA agent per kg of body weight. The length of treatment needed to observe changes and the interval following treatment for responses to occur vary depending on the desired effect. The particular amounts may be determined by conventional tests which are well known to the person skilled in the art.

As will be further detailed herein below, the pharmaceutical composition of the invention can be used, for example, to treat tumors (in particular cancer) as well as a vaccine or immunosuppressant. However, the pharmaceutical composition may also be used for the treatment or prevention of further diseases. For example, in case the protein or peptide of the invention is a fusion protein, the fusion partner may confer treatment specificity to an inflammatory disease. As discussed herein above, the use of a cytokine as fusion partner results in a fusion protein being suitable for the treatment of an inflammatory disease. Specific cytokines have been introduced into clinical trials for the treatment of stroke, Alzheimer's disease, autoimmune diseases, wound healing, and amyotrophic lateral sclerosis, one could utilize local or systemic delivery of anti-inflammatory cytokines or inflammatory cytokine antagonists for the treatment of chronic pain. As also discussed herein above, the use of a pro-coagulation factor as fusion partner results in a fusion protein being suitable for wound healing. Hence, by the fusion protein the therapeutic use of the protein or peptide of the invention can be supplemented by an additional therapeutic use conferred by the fusion partner, potentially even leading to synergistic therapeutic effects.

Herein above also radionucleides are discussed as fusion partners of the protein or peptide of the invention. A fusion protein comprising a radionucleide may be used in a radioligand therapy. In radioligand therapies the radiopharmaceuticals (usually labeled with a beta-emitter such as lutetium-177) specifically bind to a tumoral target structure (usually a surface molecule). For example, the radiopharmaceuticals may further comprise an antibody which confers specific binding to a tumoral target thereby targeting the radionucleide to the tumor. Radioligand therapies are generally well-tolerated systemic therapies used in metastasized disease. The radiopharmaceutical is generally infused or injected into a peripheral vein of the patient to be treated.

It also likely that the HLA-J nucleic acid molecule, the vector, the host cell, the protein or peptide of, the binding molecule, preferably the inhibitor of the second aspect of the invention or combinations thereof itself offer further treatment options. For instance, the HLA region encodes several molecules that play key roles in the immune system. Strong association between the HLA region and autoimmune diseases (AIDs) has been established for over fifty years (Gough and Simmons, Curr Genomics. 2007 Nov; 8(7): 453-465). Non-limiting examples of sic AIDs are type 1 diabetes (T1D), multiple sclerosis (MS), rheumatoid arthritis (RA), Graves' disease (GD), ankylosing spondylitis (AS) and systemic lupus erythematosus (SLE)

The success of bone-marrow transplantation or organ transplantation greatly depends on matching the recipient's HLA phenotype against the donor's in order to prevent graft-versus-host disease (GVHD). It has been a long debate about which HLA loci are clinically relevant. As clinical outcome data accumulates, it becomes evident that all HLA loci are relevant. It thus can be safely assumed that also HLA-J plays role. In cases where it is not possible to match the recipient's HLA-J phenotype against the donor's HLA-J phenotype the inhibition of the pharmaceutical composition of the invention could be a means for preventing GVHD.

A cosmetic composition according to the invention is for use in non-therapeutic applications. Cosmetic compositions may also be defined by their intended use, as compositions intended to be rubbed, poured, sprinkled, or sprayed on, or otherwise applied to the human body for cleansing, beautifying, promoting attractiveness, or altering the appearance. The particular formulation of the cosmetic composition according to the invention is not limited. Envisaged formulations include rinse solutions, emulsions, creams, milks, gels such as hydrogels, ointments, suspensions, dispersions, powders, solid sticks, foams, sprays and shampoos. For this purpose, the cosmetic composition according to the invention may further comprise cosmetically acceptable diluents and/or carriers. Choosing appropriate carriers and diluents in dependency of the desired formulation is within the skills of the skilled person. Suitable cosmetically acceptable diluents and carriers are well known in the art and include agents referred to in Bushell et al. (WO 2006/053613). Preferred formulations for said cosmetic composition are rinse solutions and creams. Preferred amounts of the cosmetic compositions according to the invention to be applied in a single application are between 0.1 and 10g, more preferred between 0.1 and 1g, most preferred 0.5g. The amount to be applied also depends on the size of the area to be treated and has to be adapted thereto.

As already mentioned herein above, an immunosuppressant is a drug being capable of suppressing the immune response. They can be used in immunosuppressive therapy, for example, to (i) prevent the rejection of transplanted organs and tissues (e.g., bone marrow, heart, kidney, liver), (ii) treat autoimmune diseases or diseases that are most likely of autoimmune origin (e.g., rheumatoid arthritis, multiple sclerosis, myasthenia gravis, psoriasis, vitiligo, systemic lupus erythematosus, sarcoidosis, focal segmental glomerulosclerosis, Crohn's disease, Behcet's disease, pemphigus, sclerodermia and ulcerative colitis), and/or (iii) treat non-autoimmune inflammatory diseases (e.g., long term allergic asthma control and ankylosing spondylitis).

The vaccine is preferably a tumor vaccine that either can be used to treat an existing tumor or prevent the development of a tumor. Vaccines that treat existing cancer are also known as therapeutic cancer vaccines. The vaccines may be "autologous", i.e. being prepared from samples taken from the patient, and are specific to that patient. The approach of cancer vaccination is generally to separate proteins from cancer cells and immunize patients against those proteins as antigens, with the aim of stimulating the immune system to kill the cancer cells. The antigen is in accordance with the present invention derived from HLA-J protein/peptide.

Accordingly, the present invention also relates to a method for the preparation of a tumor vaccine comprising admixing the nucleic acid molecule, the vector, the host cell, the protein or peptide, the binding molecule, preferably the inhibitor of the invention or combinations thereof with at least one pharmaceutically acceptable excipient, carrier and/or diluents.

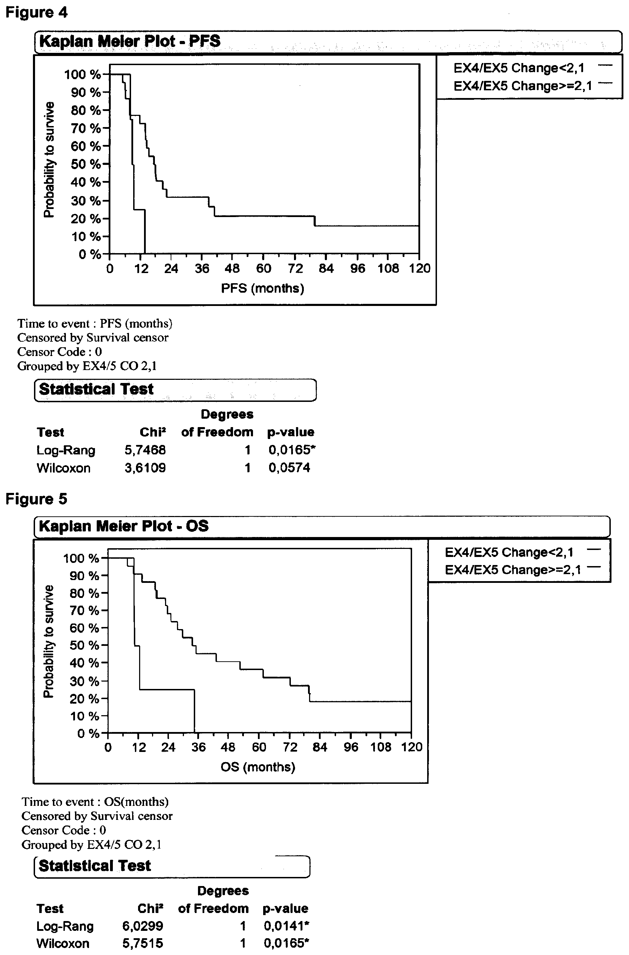

Example 2 and Figures 4/5 show that high level of HLA-J expression is associated with a significantly reduced progression free survival and overall survival of ovarian cancer patients. It thus can be safely assumed that HLA-J expression helps the tumor to escape the immune system. This in turn shows that HLA-J acts as an immunosuppressant. While the action of HLA-J as immunosuppressant is detrimental in the context of tumors it can be advantageous in the context of the above-discussed diseases which are treated in the art by immunosuppressants. This also renders it at least plausible that the vaccination of a tumor patient with HLA-J may help to suppress or abrogate the escape of the tumor from the immune system via HLA-J expression. Hence, the nucleic acid molecule, the vector, the host cell, or the protein or peptide of the invention or combinations can be used as a medicament, preferably as an immunosuppressant or as a vaccine. The nucleic acid molecule is preferably a nucleic acid molecule of item (I)(g) of the first aspect. On the other hand and as will be further detailed herein below, the binding molecule, preferably the inhibitor of the second aspect of the invention can be used, for example, to treat tumors.

A tumor is an abnormal benign or malignant new growth of tissue that possesses no physiological function and arises from uncontrolled usually rapid cellular proliferation. The tumor is preferably cancer. Cancer is an abnormal malignant new growth of tissue that possesses no physiological function and arises from uncontrolled usually rapid cellular proliferation. The cancer is preferably selected from the group consisting of breast cancer, ovarian cancer, endometrial cancer, vaginal cancer, vulvacancer, bladder cancer, salivary gland cancer, pancreatic cancer, thyroid cancer, kidney cancer, lung cancer, cancer concerning the upper gastrointestinal tract, colon cancer, colorectal cancer, prostate cancer, squamous-cell carcinoma of the head and neck, cervical cancer, glioblastomas, malignant ascites, lymphomas and leukemias. Among this list of cancers gynecologic cancers (i.e. cervical, ovarian, uterine, vaginal, and vulvar cancer) are preferred and breast cancer and ovarian cancer are most preferred. Also preferred among this list of cancers is bladder cancer.

The tumor or cancer is preferably a solid tumor or cancer. A solid tumor or cancer is an abnormal mass of tissue that usually does not contain cysts or liquid areas by contrast to a liquid tumor.

As is demonstrated herein in Example 2 and as is shown in Figures 4 and 5, high level of HLA-J mRNA expression is associated with a significantly reduced symptom free survival and overall survival of ovarian cancer patients. Similarly, it is shown in Example 3 and Figure 10 that breast cancer patients in which high levels of HLA-J were detected prior to chemotherapy are less responsive to the chemotherapy. Finally, Example 5 and Figures 18 show high level of HLA-J mRNA expression in bladder cancer patients. It follows that is was surprisingly found in connection with the present invention that the inhibition of HLA-J, e.g. the expression and/or translation of HLA-J is suitable for the treatment of tumors.

Also described herein is the use of the nucleic acid molecule of the first aspect, item (I)(g) or the protein or peptide of the first aspect in a sample obtained from a subject for diagnosing a tumor and/or for grading a tumor and/or for tumor prognosis and/or classifying tumor as a HLA-J low expression tumor or a HLA-J high expression tumor and/or for diagnosing and/or for diagnosing an implantation failure.

As mentioned, the tumor is preferably a cancer. As also mentioned, also in connection with this aspect of the invention the tumor or cancer is preferably a solid tumor or cancer.

The sample may be a body fluid of the subject or a tissue sample from an organ of the subject. Non-limiting examples of body fluids are whole blood, blood plasma, blood serum, urine, peritoneal fluid, and pleural fluid, liquor cerebrospinalis, tear fluid, or cells therefrom in solution. Non-limiting examples of tissue are colon, liver, breast, ovary, and testis. Tissue samples may be taken by aspiration or punctuation, excision or by any other surgical method leading to biopsy or resected cellular material. The sample may be a processed sample, e.g. a sample which has been frozen, fixed, embedded or the like. A preferred type of sample is a formaline fixed paraffin embedded (FFPE) sample. Preparation of FFPE samples are standard medical practice and these samples can be conserved for long periods of time.

The term "diagnosing" as used herein is directed to the identification of a disease in a subject suffering from symptoms of a disease. In accordance with the invention the disease is a tumor or an implantation failure. The term "grading" as used herein is directed to the identification of the degree of cell anaplasia of a tumor cell in a subject which has been diagnosed to have a tumor. The most commonly system used for grading tumors is the system according to the guidelines of the American Joint Commission on Cancer. As per these guidelines, the following grading categories are distinguished: GX (grade cannot be assessed), G1 (well-differentiated; low grade), G2 (moderately differentiated; intermediate grade), G3 (poorly differentiated, high grade); G4 (undifferentiated, high grade). The term "prognosis" as used herein is directed to the outlook or chance of recovery from a disease such as a tumor and/or is the outlook or chance of survival of a disease, such as a tumor. In the case of a tumor, the prognosis may comprise one or more of tumor size alteration of target lesion, disease-specific survival (DSS), recurrence-free survival (RFS), progression-free survival (PFS) and distant recurrence-free survival, wherein DSS is preferred.

The term "subject" in accordance with the invention refers to a mammal, preferably a domestic animal or a pet animal such as horse, cattle, pig, sheep, goat, dog or cat, and most preferably a human.

As discussed above, increased level of HLA-J expression in tumor patients is associated with a significantly reduced progression free survival and overall survival of tumor patients. Accordingly, higher levels of HLA-J expression also coincide with higher tumor grades. It is furthermore demonstrated in the examples that HLA-J expression was found in all tumor samples, so that HLA-J expression can not only serve as prognostic marker but also as diagnostic marker for tumors.

In the above use a positive and/or a negative sample as well as predetermined standards may be incorporated. The controls may be obtained from sample of one or more subjects, such as at least 5, at least 10, at least 20, at least 30, at least 40, at least 50, at least 60, at least 70, at least 80, at least 90, at least 100, at least 200, at least 300, at least 400, at least 500, at least 600, at least 700, at least 800, at least 900, at least 1000, at least 1500, or at least 2000 subjects. Predetermined standards designate previously obtained values from a positive and/or a negative sample(s).

For the diagnosis of a tumor it is believed that no standard at all is required since it is expected that healthy subjects do not express HLA-J. Moreover, it is shown in the examples that HLA-J expression was detected in all investigated tumor patients. Notwithstanding a positive and/or a negative sample as well as predetermined standards may be incorporated in the diagnostic tumor application of the present invention. For diagnosis the positive sample is from one or more subjects known to have a tumor, preferably a tumor of the same body site as the one to be diagnosed. Similarly, the negative sample is from one or more subjects known to have no tumor. A subject is diagnosed to have a tumor if the expression level of the nucleic acid molecule as defined in the first aspect or the protein or peptide as defined in the first aspect in a sample is with increased preference at least 1.5-fold, 2-fold, 3-fold, 4-fold increased as compared to the negative control or a predetermined standard derived therefrom. A subject is diagnosed to have a tumor if the expression level of the nucleic acid molecule as defined in the first aspect or the protein or peptide as defined in the first aspect in a sample is with increased preference less than 50%, less than 25% and less than 10% different from the positive control or a predetermined standard derived therefrom. For example, if the positive control is set to 100%, a patient displaying values of 150% to 50%, preferably 125% or 75% is diagnosed to have a tumor.

Also for the diagnosis of an implantation failure the positive and/or a negative sample as well as predetermined standards may be used. For diagnosis the positive sample is from one or more female subjects that had at least one implantation failure, preferably at least two implantation failures and most preferably at least three implantation failures. Two or more implantation failures are also referred as repetitive or recurrent implantation failure. Similarly, the negative sample is from one or more female subjects that had at least one successful pregnancy, preferably at least two successful pregnancy, and most preferably at least three successful pregnancies. A female subject is diagnosed to have an implantation failure if the expression level of the nucleic acid molecule of the first aspect or the protein or peptide as defined in the first aspect in a sample is with increased preference at least 1.5-fold, 2-fold, 3-fold, 4-fold decreased as compared to the negative control or a predetermined standard derived therefrom. A subject is diagnosed to have an implantation failure if the expression level of the nucleic acid molecule as defined in the first aspect or the protein or peptide as defined in the first aspect in a sample is with increased preference less than 50%, less than 25% and less than 10% different (i.e. higher or lower) from the positive control or a predetermined standard derived therefrom. For example, if the positive control is set to 100%, a patient displaying values of 125% or 75% is diagnosed to have an implantation failure.

With respect to classifying a tumor as a HLA-J low expression tumor or a HLA-J high expression tumor it is noted that not each and every tumor is expected to express or express substantial amounts of HLA-J. Hence, in order to reveal whether in a subject having a tumor a binding molecule, preferably an inhibitor of the second aspect of the invention can be a treatment option, the tumor may be classified as a HLA-J low expression tumor or a HLA-J high expression, wherein only in the later case the binding molecule or inhibitor is an option. For classification, the control may be from one or more subjects known to have tumor expressing HLA-J and preferably known to have tumor that was treatable by the binding molecule, preferably the inhibitor of the second aspect of the invention. In case the HLA-J expression of the tumor to be classified is with increasing preference at least 2-fold, at least 3-fold, at least 3-fold, at least 4-fold and at least 5-fold decreased as compared to the control the tumor is a HLA-J low expression tumor. On the other hand, in case the HLA-J expression of the tumor to be classified is with increasing preference at least 2-fold, at least 3-fold, at least 3-fold, at least 4-fold and at least 5-fold increased as compared to the control the tumor is a HLA-J high expression tumor.

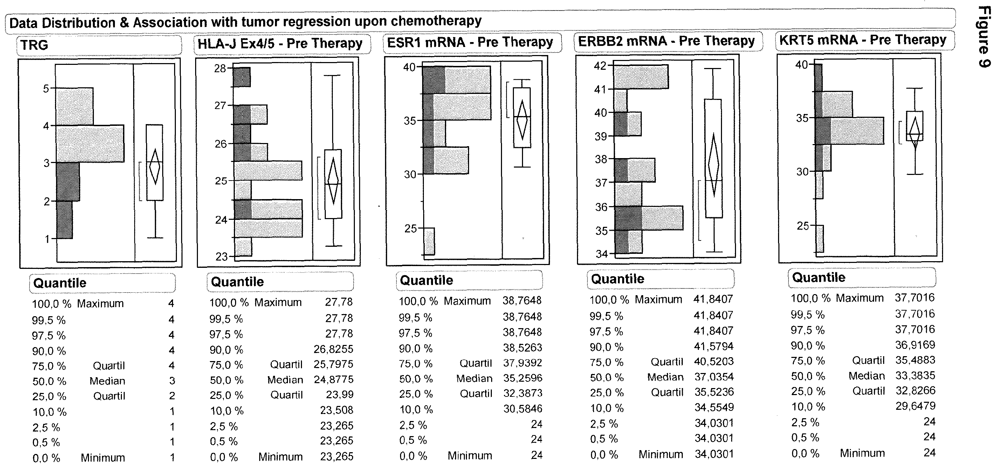

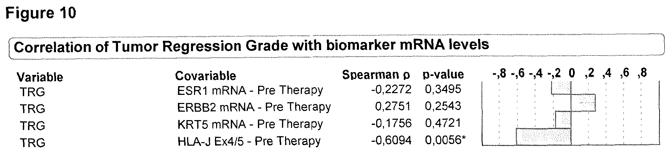

For prognosis, the positive control may be from one or more subjects that died from the tumor (preferably a tumor of the same body site as the one to be prognosed) and the negative sample may be from one or more subjects that survived the tumor for a substantial amount of time without tumor progression (preferably a tumor of the same body site as the one to be prognosed). A substantial amount designates with increased preference at least 1 year, at least 2 year, at least 3 year, at least 4 year and at least 5 years. A subject has a favorable prognosis if the expression level of the nucleic acid molecule as defined in the first aspectt or the protein or peptide as defined in the first aspect in a sample is with increased preference at least 1.5-fold, 2-fold, 3-fold, 4-fold decreased as compared to the positive control or a predetermined standard derived therefrom. Also a subject has a favorable prognosis if the expression level of the nucleic acid molecule as defined in the first aspect or the protein or peptide as defined in the first aspect in a sample is with increased preference less than 50%, less than 25% and less than 10% different from the negative control or a predetermined standard derived therefrom. A subject has an unfavorable prognosis if the expression level of the nucleic acid molecule as defined in the first aspect or the protein or peptide as defined in the first aspect in a sample is with increased preference at least 1.5-fold, 2-fold, 3-fold, 4-fold increased as compared to the negative control or a predetermined standard derived therefrom. Also a subject has an unfavorable prognosis if the expression level of the nucleic acid molecule as defined in the first aspect or the protein or peptide as defined in the first aspect in a sample is with increased preference less than 50%, less than 25% and less than 10% different from the positive control or a predetermined standard derived therefrom. With respect to the aspect of prognosis it is of note that is shown in Example 3 and Figures 9 and 10 that the levels of HLA-J expression in breast cancer patients were indicative for prognosing the response during chemotherapy. Luminal breast cancer patients had the highest HLAJ expression level and these patients were almost unresponsive to chemotherapy. Hence, the prognosis is preferably the prognosis of the expected treatment success of a tumor treatment, wherein the anti-tumor treatment is preferably chemotherapy and/or the patients to be diagnosed has preferably a breast cancer.

For grading, the positive sample may be from one or more subjects that are graded to one of the categories G1 to G4. For grading more than one positive sample can be used, wherein the positive samples are from two, preferably three and most preferably all four of categories G1 to G4. A subject is graded as having a G1 tumor if the expression level of the nucleic acid molecule as defined in the first aspect or the protein or peptide as defined in the first aspect in a sample is with increased preference less than 50%, less than 25% and less than 10% different from the positive G1 control or a predetermined standard derived therefrom. This applies *mutatis mutandis* to stages G2 to G4.

Methods for obtaining the levels of the nucleic acid molecule as defined in the first aspector the protein or peptide as defined in the first aspect as established in the art.

For instance, levels of the nucleic acid molecule as defined in the first aspect may be obtained by real time quantitative PCR (RT-qPCR), electrophoretic techniques or a DNA Microarray (Roth (2002), Curr. Issues Mol. Biol., 4: 93-100), wherein RT-qPCR is preferred. In these methods the expression level may be normalized against the (mean) expression level of one or more reference genes in the sample. The term "reference gene", as used herein, is meant to refer to a gene which has a relatively invariable level of expression on the RNA transcript/mRNA level in the system which is being examined, i.e. cancer. Such gene may be referred to as housekeeping gene. Non-limiting examples of reference genes are CALM2, B2M, RPL37A, GUSB, HPRT1 and GAPDH, preferably CALM2 and/or B2M. Other suitable reference genes are known to a person skilled in the art.

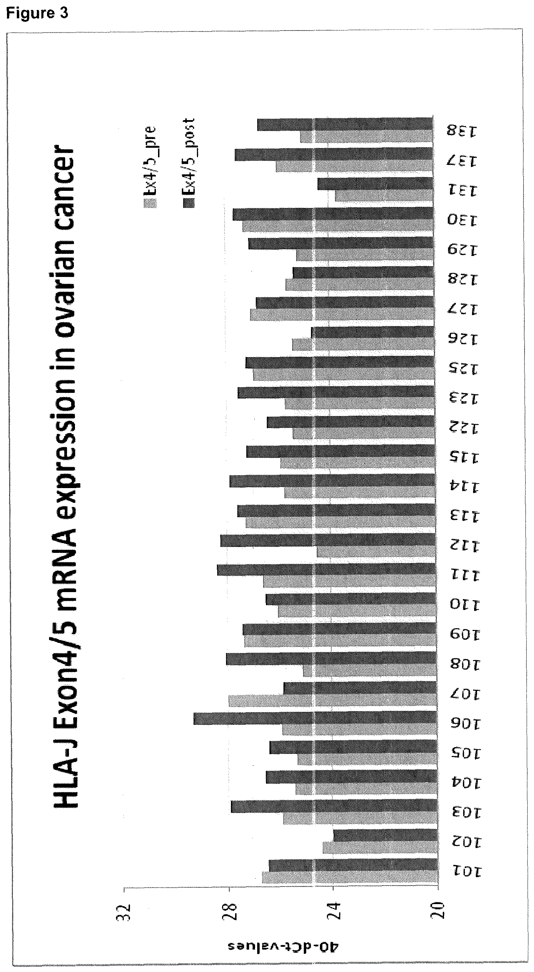

RT-qPCR is illustrated by the examples. RT-qPCR is carried out in a thermal cycler with the capacity to illuminate each sample with a beam of light of at least one specified wavelength and detect the fluorescence emitted by the excited fluorophore. The thermal cycler is also able to rapidly heat and chill samples, thereby taking advantage of the physicochemical properties of the nucleic acids and DNA polymerase. The two common methods for the detection of PCR products in real-time qPCR are: (1) non-specific fluorescent dyes that intercalate with any double-stranded DNA, and (2) sequence-specific DNA probes consisting of oligonucleotides that are labelled with a fluorescent reporter which permits detection only after hybridization of the probe with its complementary sequence (e.g. a TaqMan probe). The latter detection method is used in the examples herein below. The probes are generally fluorescently labeled probes. Preferably, the fluorescently labeled probe consists of an oligonucleotide labeled with both a fluorescent reporter dye and a quencher dye (= dual-label probe). Suitable fluorescent reporter and quencher dyes/moieties are known to a person skilled in the art and include, but are not limited to the reporter dyes/moieties 6-FAMTM, JOETM, Cy5^{®}, Cy3^{®} and the quencher dyes/moieties dabcyl, TAMRATM, BHQTM-1, -2 or - 3. Preferably primers for use in accordance with the present invention have a length of 15 to 30 nucleotides, and are in particular deoxyribonucleotides. In one embodiment, the primers are designed so as to (1) be specific for the target mRNA-sequence of HLA-J or being derived therefrom, (2) provide an amplicon size of less than 120 bp (preferably less than 100 bp), (3) be mRNA-specific (consideration of exons/introns; preferably no amplification of genomic DNA), (4) have no tendency to dimerize and/or (5) have a melting temperature Tₘ in the range of from 58°C to 62°C (preferably, Tₘ is approximately 60°C). More preferably one or more of the primers and probes specifically bind to the HLA-J specific Exon 4 / Exon 5 junction of HLA-J (Fig 3). Even more preferably one or more of the primers and probes as shown in Table 1 of the examples are used in a RT-qPCR, more preferably one of the specific primers pairs which can optionally be used in connection with the respective probe. As mentioned, the probe is required for a RT-qPCR according to (2) but the probe can be replaced by an intercalating dye in the case of a RT-qPCR according to (1), such as SYBR green. The forward primers of Table 1 correspond to SEQ ID NOs 12 to 25, the probes to SEQ ID NOs 26 to 39 and the reverse primers to SEQ ID NOs 40 to 53. The forward primer of SEQ ID NO: 12 and reverse primer of SEQ ID NO: 40 are a primer pair and SEQ ID NO: 26 is the corresponding probe, the forward primer of SEQ ID NO: 13 and reverse primer of SEQ ID NO: 41 are a primer pair and SEQ ID NO: 27 is the corresponding probe, etc. Most preferably a primer pair of SEQ ID NOs 18 and 46, optionally along with the probe of SEQ ID NO: 32 is used. This primer pair and probe are specific for the HLA-J specific Exon 4 / Exon 5 junction of HLA-J.

As an alternative of qPCR also electrophoretic techniques or a DNA microarray may be used to obtaining the levels of the nucleic acid molecule as defined in the first aspect of the invention. The conventional approach to mRNA identification and quantitation is through a combination of gel electrophoresis, which provides information on size, and sequence-specific probing. The Northern blot is the most commonly applied technique in this class. The ribonuclease protection assay (RPA) was developed as a more sensitive, less labor-intensive alternative to the Northern blot. Hybridization is performed with a labeled ribonucleotide probe in solution, after which non-hybridized sample and probe are digested with a mixture of ribonucleases (e.g., RNase A and RNase T1) that selectively degrade single-stranded RNAs. Subsequent denaturing polyacrylamide gel electrophoresis provides a means for quantitation and also gives the size of the region hybridized by the probe. For both Northern blot and RPA, the accuracy and precision of quantitation are functions of the detection method and the reference or standard utilized. Most commonly, the probes are radiolabeled with 32P or 33P, in which case the final gel is exposed to X-ray film or phosphor screen and the intensity of each band quantified with a densitometer or phosphor imager, respectively. In both cases, the exposure time can be adjusted to suit the sensitivity required, but the phosphorbased technique is generally more sensitive and has a greater dynamic range. As an alternative to using radioactivity, probes can be labeled with an antigen or hapten, which is subsequently bound by a horseradish peroxidase- or alkaline phosphatase-conjugated antibody and quantified after addition of substrate by chemiluminesence on film or a fluorescence imager. In all of these imaging applications, subtraction of the background from a neighboring region of the gel without probe should be performed. The great advantage of the gel format is that any reference standards can be imaged simultaneously with the sample. Likewise, detection of a housekeeping gene is performed under the same conditions for all samples.

For the construction of DNA microarrays two technologies have emerged. Generally, the starting point in each case for the design of an array is a set of sequences corresponding to the genes or putative genes to be probed. In the first approach, oligonucleotide probes are synthesized chemically beginning from a glass substrate. Because of the variable efficiency of oligonucleotide hybridization to cDNA probes, multiple oligonucleotide probes are synthesized complementary to each gene of interest. Furthermore, for each fully complementary oligonucleotide on the array, an oligonucleotide with a mismatch at a single nucleotide position is constructed and used for normalization. Oligonucleotide arrays are routinely created with densities of about 10⁴-10⁶ probes/cm². The second major technology for DNA microarray construction is the robotic printing of cDNA probes directly onto a glass slide or other suitable substrate. A DNA clone is obtained for each gene of interest, purified, and amplified from a common vector by PCR using universal primers. The probes are robotically deposited in spots on the order of 50-200 µm in size. At this spacing, a density of, for example, approximately 10³ probes/cm² can be achieved.

Levels of the protein or peptide as defined in the first aspect may be determined, for example, by using a "molecule binding to the protein or peptide" and preferably a "molecule specifically binding to the protein or peptide". A molecule binding to the protein or peptide designates a molecule which under known conditions occurs predominantly bound to the protein or peptide. A "molecule binding to the protein or peptide" one of the herein above described binding molecules, preferably inhibitors of the protein or peptide as defined in the first aspectmay be used, such as antibodies, aptamers, etc. Levels of the protein or peptide as defined in the first aspect may also be obtained by using Western Blot analysis, mass spectrometry analysis, FACS-analysis, and ELISA. These techniques are non-limiting examples of methods which may be used to qualitatively, semi-quantitatively and/or quantitatively detect a protein or peptide.

Western blot analysis is a widely used and well-know analytical technique used to detect specific proteins or peptides in a given sample, for example, a tissue homogenate or body extract. It uses gel electrophoresis to separate native or denatured proteins or peptides by the length of the (poly)peptide (denaturing conditions) or by the 3-D structure of the protein (native/ non-denaturing conditions). The proteins or peptides are then transferred to a membrane (typically nitrocellulose or PVDF), where they are probed (detected) using antibodies specific to the target protein.

Also mass spectrometry (MS) analysis is a widely used and well-know analytical technique, wherein the mass-to-charge ratio of charged particles is measured. Mass spectrometry is used for determining masses of particles, for determining the elemental composition of a sample or molecule, and for elucidating the chemical structures of molecules, such as proteins, peptides and other chemical compounds. The MS principle consists of ionizing chemical compounds to generate charged molecules or molecule fragments and measuring their mass-to-charge ratios.

Fluorescence activated cell sorting (FACS) analysis is a widely used and well-known analytical technique, wherein biological cells are sorted based upon the specific light scattering of the fluorescent characteristics of each cell. Cells may be fixed in 4% formaldehyde, permeabilized with 0.2 % Triton-X-100, and incubated with a fluorophore-labeled antibody (e.g. mono- or polyclonal anti-HLA-J antibody).

Enzyme-linked immunosorbent assay (ELISA) is a widely used and well-know sensitive analytical technique, wherein an enzyme is linked to an antibody or antigen as a marker for the detection of a specific protein or peptide.

Next to the nucleic acid molecule as defined in the first aspect or the protein or peptide as defined in the first aspect one or more further compounds in the sample obtained from a subject may be used for diagnosing a tumor and/or for grading a tumor and/or for tumor prognosis. A vast number of markers for diagnosing a tumor and/or for grading a tumor and/or for tumor prognosis are known in the art may be used in conjunction with the nucleic acid molecule as defined in the first aspect or the protein or peptide as defined in the first aspect. Several tumor markers are indicative for particular tumor, such as breast cancer or colon cancer. Tumor markes are, for example listed at the National Cancer Institute (https://www.cancer.gov/about-cancer/diagnosis-staging/diagnosis/tumor-markers-fact-sheet) or the integrated database of cancer genes and markers CGMD (http://cgmd.in/). The use of one or more further markers generally increases the reliability of the diagnosis, the grading or the prognosis.

In an third aspect the invention relates to a method for diagnosing a tumor comprising detecting the presence of the nucleic acid molecule as defined in the first aspect, item (l)(g) and/or the protein or peptide as defined in the first aspect in a sample obtained from a subject, wherein the presence of the nucleic acid molecule as defined in the first aspect, item (l)(g) and/or the protein or peptide according to the invention is indicative for a tumor in the subject.

In a fourth aspect the invention relates to a method for grading a tumor and/or for tumor prognosis comprising determining the level of the nucleic acid molecule as defined in the first aspect, item (l)(g) and/or the protein or peptide as defined in the first aspect in a sample obtained from a subject, wherein increased levels of the nucleic acid molecule of the first aspect, item (I)(g) and/or the protein or peptide as defined in the first aspect as compared to a control positively correlate with the a higher grade of the tumor and/or an adverse tumor prognosis.

The methods of the third and fourth aspect of the invention implement the above-described use for diagnosing a tumor and/or for grading a tumor and/or for tumor prognosis and/or classifying tumor as a HLA-J low expression tumor or a HLA-J high expression tumor and/or for diagnosing and/or for diagnosing an implantation failure in the format of methods. It follows that the definitions and preferred embodiments provided herein above in connection with this use are equally applicable to the eleventh and twelfth aspect of the invention.

In a fifth aspect the invention relates to a method for monitoring the non-efficacy of a tumor treatment in a subject having a tumor comprising (a) determining the amount of the nucleic acid molecule as defined in the first aspect, item (l)(g) and/or the protein or peptide as defined in the first aspect in a sample obtained from a subject before the start of the treatment; and (b) determining the amount of the nucleic acid molecule as defined in the first aspect, item (l)(g) and/or the protein or peptide as defined in the first aspect in a sample obtained from a subject at one or more times after the start of the treatment, wherein an increased amount in b) as compared to a) is indicative for the non-efficacy of a tumor treatment and/or decreased amount in b) as compared to a) is indicative for the efficacy of a tumor treatment.

Similarly, in a sixth aspect the invention relates to a method for monitoring the non-efficacy of a immunosuppressive therapy in a subject requiring such a therapy comprising (a) determining the amount of the nucleic acid molecule as defined in the first aspect, item (l)(g) and/or the protein or peptide as defined in the first aspect in a sample obtained from a subject before the start of the therapy; and (b) determining the amount of the nucleic acid molecule as defined in the first aspect, item (l)(g) and/or the protein or peptide as defined in the first aspect in a sample obtained from a subject at one or more times after the start of the therapy, wherein a decreased amount in b) as compared to a) is indicative for the non-efficacy of a immunosuppressive therapy and/or an increased amount in b) as compared to a) is indicative for the efficacy of a immunosuppressive therapy.

The definitions and preferred embodiments provided herein above in connection with the other aspects of the invention are equally applicable to the fifth and sixth aspect of the invention. For example, means and methods for determining the amount of the nucleic acid molecule as defined in the first aspect, item (l)(g) and/or the protein or peptide as defined in the first aspect have been described herein above in connection with the the above-described use for diagnosing a tumor and/or for grading a tumor and/or for tumor prognosis and/or classifying tumor as a HLA-J low expression tumor or a HLA-J high expression tumor and/or for diagnosing and/or for diagnosing an implantation failure. These means and methods can equally be used in connection with the fourteenth and fifteenth aspect of the invention.

The tumor treatment can be any tumor treatment, for example, surgery, radiotherapy or chemotherapy. The tumor treatment is preferably chemotherapy. Chemotherapy comprises the administration of chemotherapeutic agents. Chemotherapeutic agents that can be used according to the invention include cytostatic compounds and cytotoxic compounds. Traditional chemotherapeutic agents act by killing cells that divide rapidly, one of the main properties of most cancer cells. Chemotherapeutic agents include but are not limited to taxanes, nucleoside analogs, camptothecin analogs, anthracyclines and anthracycline analogs, etoposide, bleomycin, vinorelbine, cyclophosphamide, antimetabolites, anti-mitotics, and alkylating agents. The chemotherapy may also be platinum-based, i.e. comprises the administration of platinum-based compounds, e.g., cisplatin. Chemotherapeutic agents are often given in combinations, usually for 3-6 months. One of the most common treatments comprises the administration of cyclophosphamide plus doxorubicin (adriamycin; belonging to the group of anthracyclines and anthracycline analogs), known as AC. Sometimes, a taxane drug, such as docetaxel, is added, and the regime is then known as CAT; taxane attacks the microtubules in cancer cells. Another common treatment, which produces equivalent results, comprises the administration of cyclophosphamide, methotrexate, which is an antimetabolite, and fluorouracil, which is a nucleoside analog (CMF). Another standard chemotherapeutic treatment comprises the administration of fluorouracil, epirubicin and cyclophosphamide (FEC), which may be supplemented with a taxane, such as docetaxel, or with vinorelbine.

The tumor is in accordance with the fifth aspect, preferably a non-luminal tumor. A non-luminal is a hormone-receptor (oestrogen-receptor and/or progesterone-receptor) negative tumor or a tumor expressing a low level of hormone-receptor (oestrogen-receptor and/or progesterone-receptor). In the case of breast tumors, luminal A tumors are hormone-receptor positive, Her2 negative, and express low levels of Ki-67, and luminal B tumors are (i) hormone-receptor positive, Her2 negative, and express high levels of Ki-67, or (ii) are oestrogen-receptor positive, progesterone-receptor negative, Her2 negative, and express low levels of Ki-67. The non-luminal breast tumors can be devided into HER2 positive tumors and TNBC (triple negative breast cancer), being HER2 negative and hormone-receptor (oestrogen-receptor and/or progesterone-receptor) negative.

Likewise the immunosupressive therapy can be any immunosupressive therapy. For example, the immunosupressive therapy may comprise the administration of one or more immunosuppressive drugs, e.g. selected from glucocorticoids, cytostatics and antibodies. In accordance with the fifth aspect, the subject may have received a transplanted organ or tissue (e.g., bone marrow, heart, kidney, liver), or may have an autoimmune diseases or a disease that is most likely of autoimmune origin (e.g., rheumatoid arthritis, multiple sclerosis, myasthenia gravis, psoriasis, vitiligo, systemic lupus erythematosus, sarcoidosis, focal segmental glomerulosclerosis, Crohn's disease, Behcet's Disease, pemphigus, ankylosing spondylitis, and ulcerative colitis) or another non-autoimmune inflammatory diseases (e.g., long term allergic asthma control, or ankylosing spondylitis).

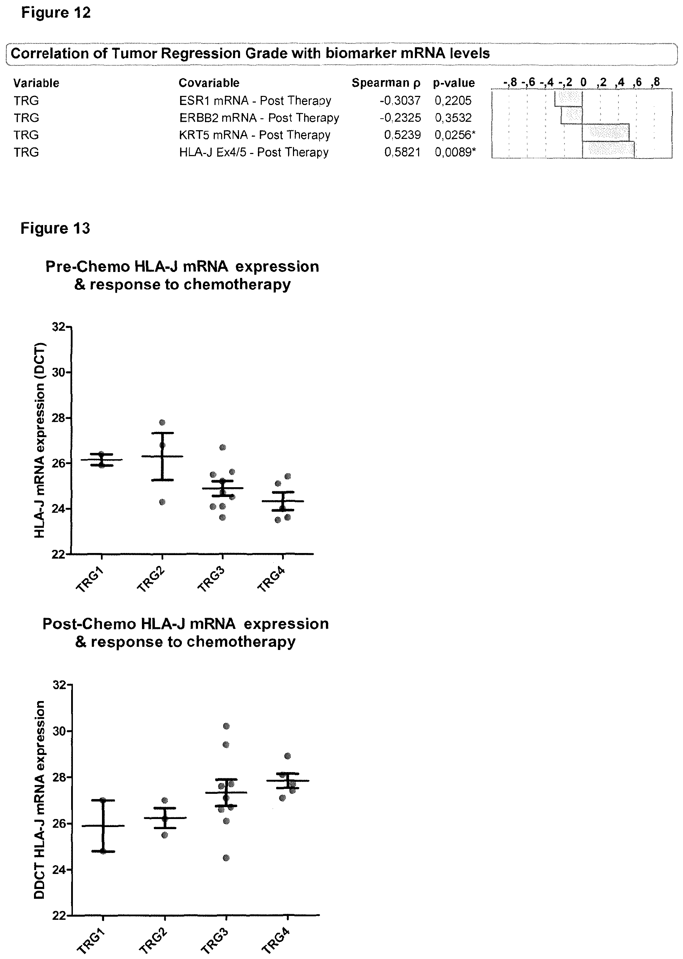

In Example 2 and Figures 4 and 5 an 8-fold increase of HLA-J expression upon exposure of ovarian cancer tissue to chemotherapy is demonstrated. Similarly, in breast cancer patients a significant upregulation of HLA-J expression during chemotherapy was found to be positively correlated with a subtotal response to the chemotherapy; see Example 3 and Figure 12. The higher the increase of HLA-J expression the worse was the progression free and overall survival of the respective tumor patient. In particular, an at least 2-fold, preferably at least 3-fold, more preferably at least 4-fold, even more preferably at least 6-fold and most preferably an at least 8-fold increase is indicative for a worse prognosis.

The Figures show.
**Fig 1****.** Sequence alignment of HLA-A1,HLA-A2, HLA-G, HLA-F1, HLA-F2 and HLA-F3 at the potential translation initiation site. Potential start codons are depicted by light gray background. Differences at the start codons with the published consensus sequence are marked with yellow background. Splice sites are depicted by gray background. The described premature stop codon of HLA-J is marked with gray background and gray letters.
**Fig 2****.** Sequence alignment of HLA-A1,HLA-A2, HLA-G, HLA-F1, HLA-F2 and HLA-F3 at transition into the transmembrane domain. Splice sites are depicted by gray background. The prolonged spanning between Exon 5 - Exon 6 donor acceptor sites stems from the insertion of the HLA-J unique sequence. The hereby disclosed new stop codon of HLA-J is marked with gray background and gray letters.
**Fig 3****.** HLA-J mRNA expression as quantified by RT-qPCR assay at the HLA-J specific Exon 4 / Exon 5 junction. Relative mRNA expression is determined by the 40-DCT method using CALM2 as reference gene. The higher the 40-DCT value, the higher the gene expression. Due to exponetntial nature of the PCR method each increase by 1 means a doubling of gene expression. An increase of 3 DCT values means an 8 fold increase of HLA-J mRNA expression.
**Fig 4****.** Kaplan Meier Plot displaying progression free survival probability of neoadjuvantly treated ovarian cancer patients based on stratification by HLA-J mRNA expression as quantified by RT-qPCR assay at the HLA-J specific Exon 4 / Exon 5 junction. Relative mRNA expression is determined by the 40-DCT method using CALM2 as reference gene. Differences between pre- and post-treatment expression levels were determined on the basis of 40-DCT calculation (see above). A more than 2 DCT change (i.e. >= 2.1) is reflecting a > 8 fold increase of HLA-J upon exposure of ovarian cancer tissue to chemotherapy.
**Fig 5****.** Kaplan Meier Plot displaying overall survival probability of neoadjuvantly treated ovarian cancer patients based on stratification by HLA-J mRNA expression as quantified by RT-qPCR assay at the HLA-J specific Exon 4 / Exon 5 junction. Relative mRNA expression is determined by the 40-DCT method using CALM2 as reference gene. Differences between pre- and post-treatment expression levels were determined on the basis of 40-DCT calculation (see above). A more than 2,1 DCT change is reflecting a > 8 fold increase of HLA-J upon exposure of ovarian cancer tissue to chemotherapy.

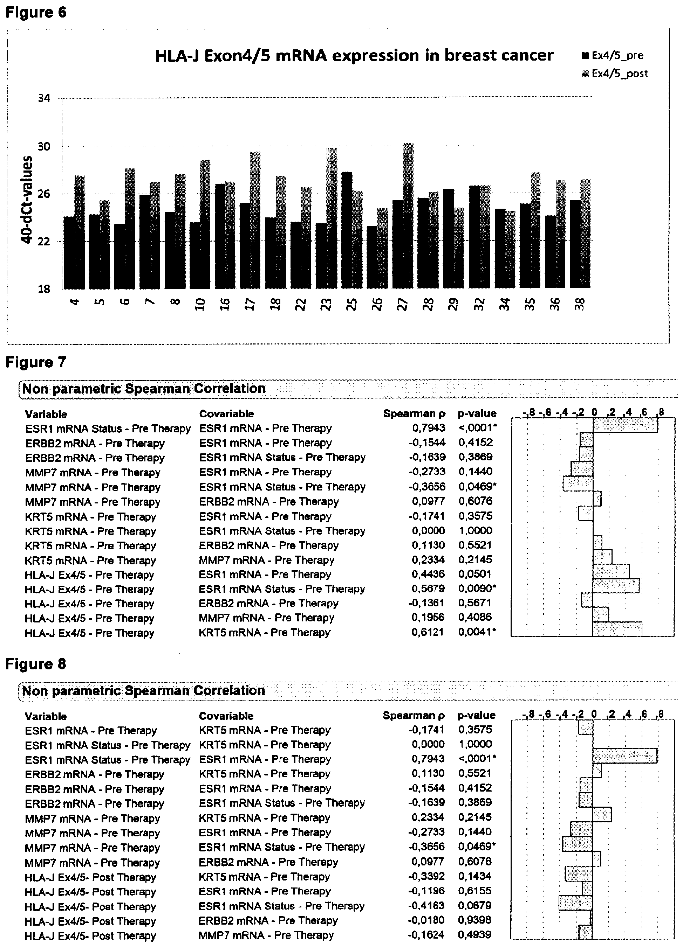

Fig 6. HLA-J mRNA expression as quantified by RT-qPCR assay at the HLA-J specific Exon 4 / Exon 5 junction. Relative mRNA expression is determined by the 40-DCT method using CALM2 as reference gene. The higher the 40-DCT value, the higher the gene expression.
Fig 7. Correlation of HLA-J mRNA expression in chemotherapy naïve pretreatment core needle biopsies of breast cancer tissues with key markers of breast cancer subtyping.
Fig 8. Correlation of HLA-J mRNA expression in surgical specimen of remaining breast cancer tissues after chemotherapy treatment with key markers of breast cancer subtyping.
Fig 9. Data distribution of Tumor Regression Grade (TRG) status and mRNA expression levels of HLA-J, ESR1, ERBB2 and KRT5 mRNA in pretreatment core needle biopsy samples from breast cancer patients.
Fig 10. Spearman correlation of TRG status and mRNA expression levels of HLA-J, ESR1, ERBB2 and KRT5 mRNA in pretreatment core needle biopsy samples from breast cancer patients.

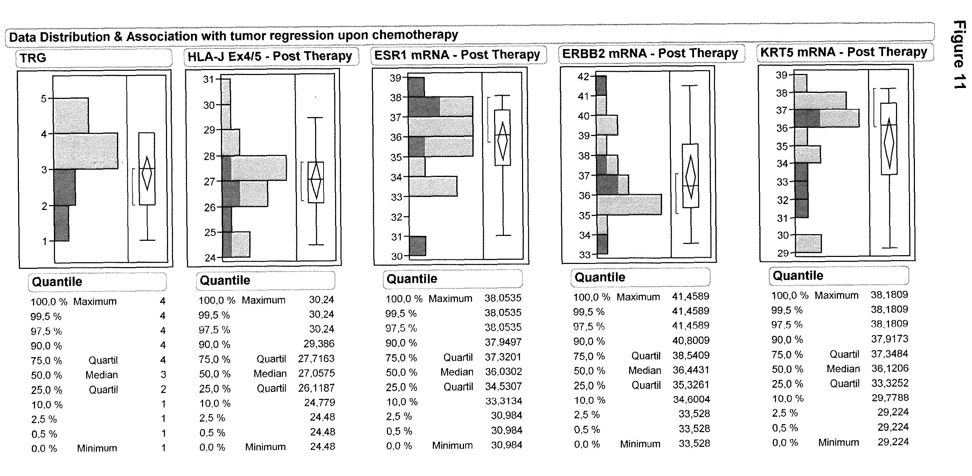

Fig 11. Data distribution of TRG status and mRNA expression levels of HLA-J, ESR1, ERBB2 and KRT5 mRNA of breast tumors after neoadjuvant chemotherapy. Tumor tissues with limited response to neoadjuvant chemotherapy are highlighted in dark gray.
Fig 12. Spearman correlation of TRG status and mRNA expression levels of HLA-J, ESR1, ERBB2 and KRT5 mRNA in tumor tissues from breast cancer patients after neoadjuvant chemotherapy.
Fig 13. Scatter plot of HLA-J mRNA expression (y-axis) according to TRG status (x-axis) in tumor tissues from breast cancer patients before (left panel) and after (right panel) neoadjuvant chemotherapy.

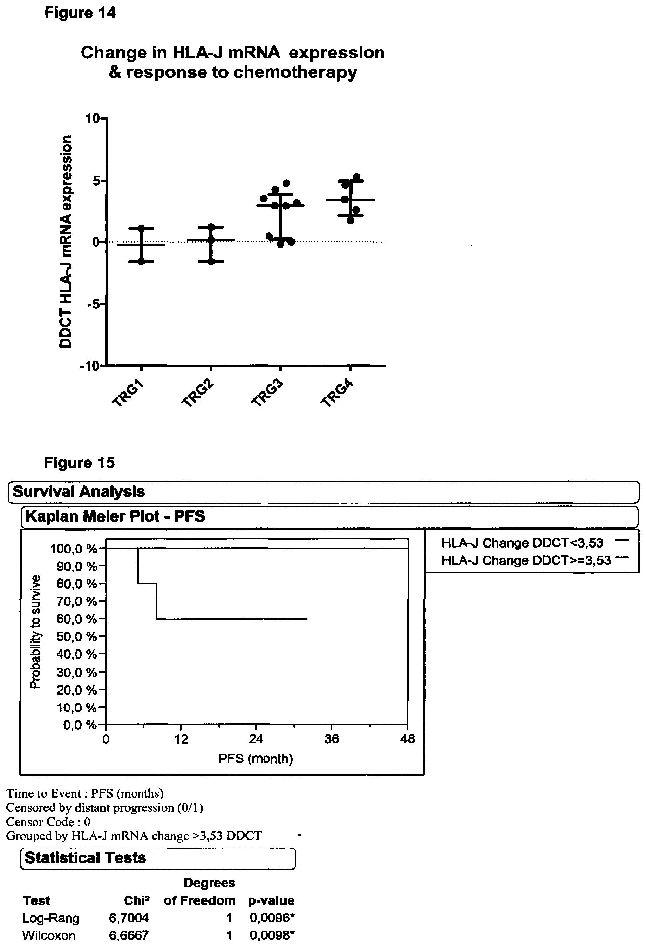

Fig 14. Scatter plot of HLA-J mRNA expression differences before and after chemotherapy (y-axis) according to TRG status (x-axis) in tumor tissues from breast cancer patients.
Fig 15. Kaplan Meier Plot displaying progression free survival probability of neoadjuvantly treated breastcancer patients based on stratification by HLA-J mRNA expression as quantified by RT-qPCR assay at the HLA-J specific Exon 4 / Exon 5 junction. Relative mRNA expression is determined by the 40-DCT method using CALM2 as reference gene. Difference between pre- and post-treatment expression levels were determined on basis of 40-DCT calculation (see above). A more than 3,5 DCT change reflecting a > 16 fold increase of HLA-J upon exposure of breast cancer tissue to chemotherapy.

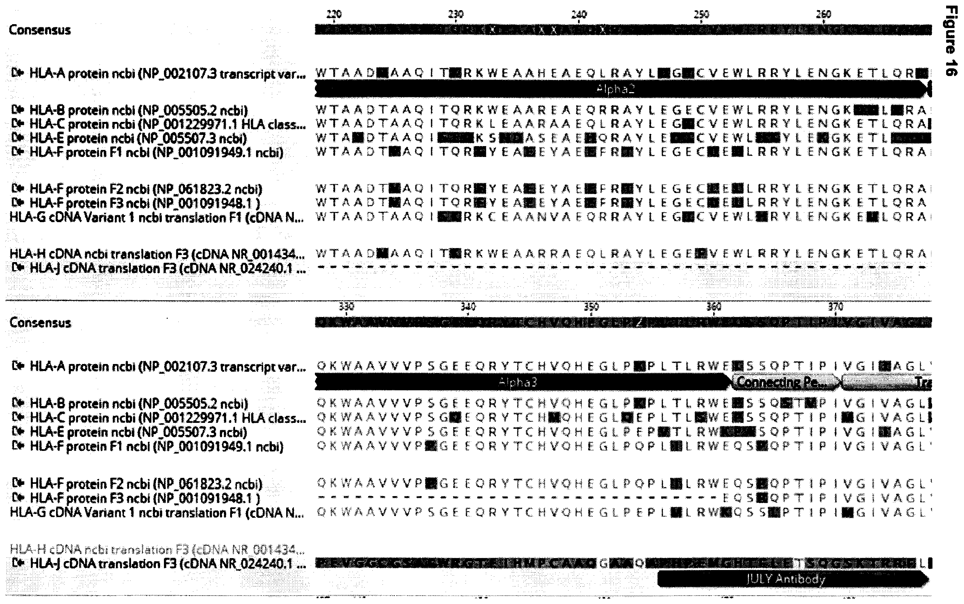

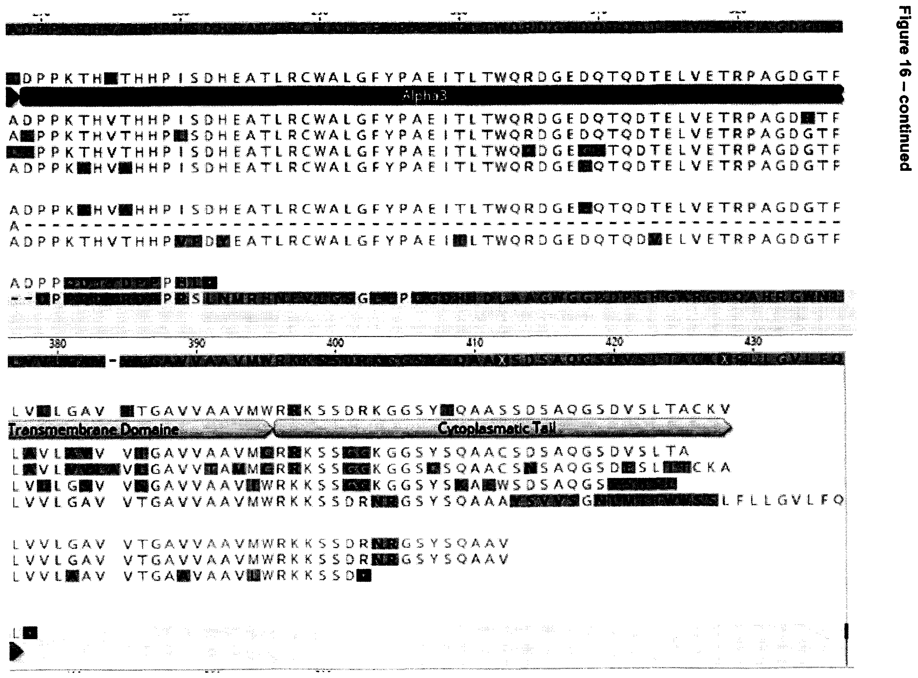

Fig 16. Summary of the protein sequences of HLA-A, HLA-B, HLA-C, HLA-E, HLA-F isoforms, HLA-G, HLA-H and HLA-J in the region of the alpha 2 and 3 domains, the transmembrane domains and the corresponding connecting peptide with the cytoplasmatic region. The consensus sequence is highlighted in grey above the aligned sequences. Differences in the HLA peptide sequences are also highlighted in grey. The predicted alpha 3 domain of HLA-J differs from these doaim of the other HLA genes and is highlighted in grey. The peptide sequence for the generation of a HLA-J specific antibody is depicted as arrow, named "JULY Antibody".

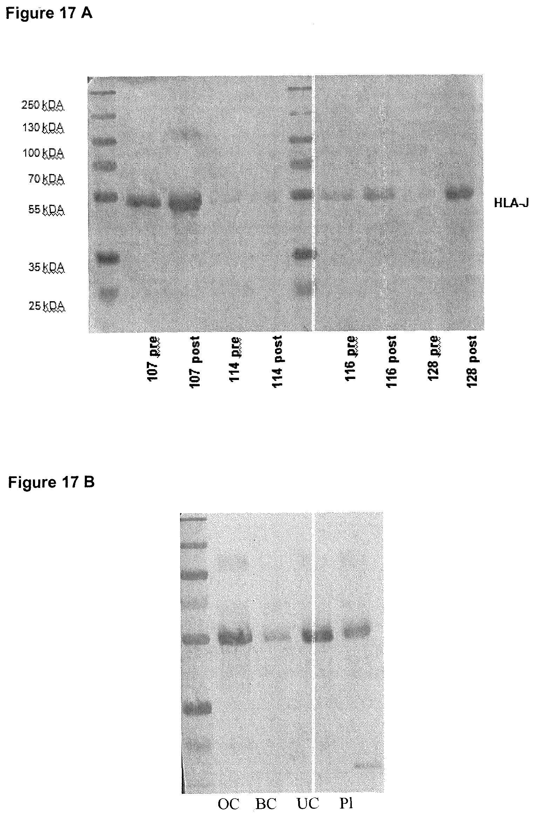

Fig 17. (A) Evidence of HLA-J protein expression by western blot analysis in ovarian cancer tissue from patients (n=4; patient ID: 107, 114, 116 and 128)), which suffered from chemo therapy resistant tumors. For each patient, HLA-J protein expression has been evaluated before (pre) and after (post) neoadjuvant chemotherapy. (B) Evidence of HLA-J protein expression by western blot analysis in ovarian cancer ("OC"), breast cancer ("BC"), urothelial/blacker cancer ("UC") and plazental tissue ("PI").

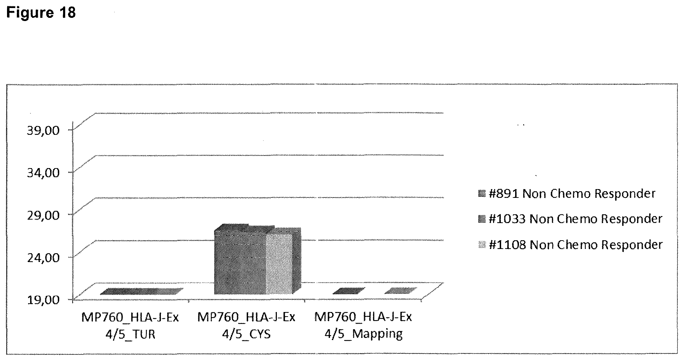

Fig 18. Tumor-specific expression of HLA-J in cystectomy specimen (CYS) biopsy samples from three cancer patients after neoadjuvant chemotherapy, while no expression was detected in the matched transurethral resection (TUR) biopsy samples before chemotherapeutic treatment as well as in "mapping" negative control samples from normal tissue.

The examples illustrate the invention.

### Example 1: Identification of HLA-J as a non pseudogene and determination of an HLA-J specific region at respective Exon/Exon boundary.

Genome Analysis and Sequence alignment were done by accessing UCSC genome browser (https://genome.ucsc.edu/cgi-bin/hgGateway) and downloading the genomic sequences of HLA-A1 (NM_002116.7), HLA-A2 (NM_001242758.1), HLA-G (NM_002127.5), HLA-F1 (NM_001098479.1), HLA-F2 (NM_018950.2), HLA-F3 (NM_001098478.1) with the putative sequence of HLA-J (NR_024240.1). The initial alignment analysis focused on the potential translation initiation region and the potential transition from extracellular alpha domains into the transmembrane region. According to previous publication of Messer et al. (Messer G, Zemmour J, Orr HT, Parham P, Weiss EH, Girdlestone J. HLA-J, a second inactivated class I HLA gene related to HLA-G and HLA-A. Implications for the evolution of the HLA-A-related genes. J Immunol. 1992 Jun 15; 148(12):4043-53.) HLA-J is thought to be a pseudogene, because of deleterious mutations that produce translation termination either in exon 2 or exon 4. To analyze potential reading frames the start codon was analyzed, which is the first codon of a messenger RNA (mRNA) transcript translated by a ribosome. Messer et al. based their sequence interpretation on a consensus start site as follows ATG-GGG-GTC-ATG-GCG-CCC-CGA-ACC-C (SEQ ID NO: 10) (Fig. 1, ATG with light gray background in the HLA-G Sequence). According to their sequence analysis of the HLA-J cloned by their group a deletirous point mutation can be observed at nucleotide position 4, where a G is missing (Fig. 1, gray "-" in the HLA-J Sequence), which indeed results in a frame shift ATG-GGG-TCA-TGG-CGC-CCC-GAA-CCC (SEQ ID NO: 11). As described by Messer et al., this results in an early termination of the protein sequence in exon 2. However, the further research described herein surprisingly revealed that there is a second potential start site at position 10. Sequence analysis revealed that the surrounding nucleotides of the second ATG at 5' and 3' end are in line with the requirements of a Kozak Sequence. Moreover, when comparing the published start codon consensus, HLA-F misses the consensus start codon as described by Messer et al., but does have the second start site, which according to the present invention, is the true translations initiation site for HLA-J. The sequence alignments were analyzed and it was found that HLA-J when starting at position 10 and having the translation initiation codon at the second ATG site does not have a frame shift deletion resulting in any premature stop, but a protein substantially similar to HLA-G. "Open Reading Frame 3" (ORF3) of the consensus alignment was identified to be the relevant reading frame, which turned the published "pseudogene" into a true gene sequence.

Sequence alignment of HLA-A1, HLA-A2, HLA-G, HLA-F1, HLA-F2, HLA-F3 and HLA-J at the transition from the alpha domain into the transmembrane domain is depicted in Figure 2. Based on the NCBI Nomenclature exon 5 codes for the alpha 23 domain of HLA-s and exon 6 codes for the transmembrane domain. When looking at the spice site at the conjunction between exon 5 and exon 6 (depicted by gray background colour in Fig. 2 according to the HLA-G sequence in gray letters) it becomes evident, that an insertion of 16 nucleotides within HLA-J destroys this splicing donor and acceptor site, while creating a sequence, which is unique for HLA-J. No other HLA does have a similar sequence at this position or anywhere else in the whole sequence. Importantly this region stems from a true exonic region, with an intron length of approximately 570 bp between the HLA-J specific donor and acceptor sites for splicing. Therefore this region was selected to be of particular interest to prove existence of secreted HLA-J mRNA in tumor and normal tissue samples (see example 2).

When comparing the exon structure of the non pseudogene HLA-J with HLA-G, it becomes clear, that HLA-J particularly lacks an exon 4 homolog coding for the alpha domain structure 2 and functions as a genomically determined splice variant homolog of HLA-G. Even more importantly, the deduced amino acid sequence based on ORF3, i.e. "HTFLETSQGSKTRRFL(-Stop)" (SEQ ID NO: 5) (contains multiple charged amino acids (such as E, K, R) and aromatic amino acids (F), which excludes this sequences to be a transmembrane spanning region or anchor. Thereby the newly identified HLA-J is clearly a secreted protein with features similar to HLA-G but lacking an alpha 2 like domain, which would be important for peptide presentation.

### Example 2: Determination of HLA-J mRNA expression levels by reverse transcription (RT) quantitative PCR (RT-qPCR) in a neoadjuvantly treated ovarian cancer patient cohort

Forty-five newly diagnosed patients with histologically confirmed FIGO stage III-IV epithelial ovarian or peritoneal carcinoma unsuitable for optimal upfront surgery and candidate for neo-adjuvant chemotherapy (said carcinoma also referred to herein below as ovarian cancer) were enrolled in the study between September 2004 and December 2007. Other inclusion criteria were age >18 years, haematological, renal, hepatic and cardiac function adequate for platinum-based chemotherapy. Exclusion criteria were a Karnofsky performance status (KPS) lower than 70%, a history of other malignancies and contraindications for surgery. The possibility of optimal debulking surgery was excluded at baseline by open laparoscopy. The initial study population of 45 patients was restricted to 35, after excluding nine patients whose biopsy samples were not adequate for the microarray analysis and one patient found to be ineligible because of diagnosis of peritoneal mesothelioma after histological revision. A standard regimen of carboplatin AUC 5 and paclitaxel 175 mg/m2 Q3 over 3 h every 3 weeks was administered as neo-adjuvant treatment for six cycles. In three patients older than 75 years and in one patient with poor performance status (KPS 70%), single-agent carboplatin was preferred to the combination chemotherapy.

Histopathological response was evaluated after surgery, with surgical samples analysis. To date, no histopathological criteria have been firmly established to describe treatment response after neo-adjuvant chemotherapy in ovarian cancer. According to the literature concerning response to primary chemotherapy in ovarian (Le et al. 2007, Sassen et al. 2007) and breast cancer (Ogston et al. 2003), as complete pathological response the absence of cancer cells in surgical specimens, and as very good partial remission the persistence of only small clusters (<1 cm) or individual cancer cells and no macroscopic residual after surgery was considered. Partial pathological remission was defined as a tumor burden reduction between 30% and 90% at surgery, while stable disease was defined as no tumor burden reduction or reduction lower than 30% at surgery, compared with initial diagnostic laparoscopy. Only patients with complete and very good partial remissions were considered as pathological responders, while all the other cases were considered as pathological non-responders. For survival analysis, the time from initial diagnosis until progression (PFS) or until death (OS) or the time between progression and death (PDT) were used for Kaplan Meier survival estimates and cox regression analysis. Complete survival data were available from 40 patients. At time of data closure, the median PFS was at 14.7 months and the median OS was at 33.5 months, which is high given the very advanced stage of the disease at study entry (unresectable FIGO III - IV).

For mRNA detection, tissues collected were snap frozen and stored in liquid nitrogen until analysis. Approximately 20-100 mg of frozen ovarian tumor tissue was crushed in liquid nitrogen. RNA was extracted using commercial kits (Qiagen), RNA integrity was assessed on the Agilent 2100 Bioanalyzer (Agilent Technologies, Palo Alto, CA, USA), cDNA was synthesized from 1 mg of total RNA using Invitrogen kits (Invitrogen Corp.) and analysed on Affymetrix HG-U133A microarrays (Affymetrix Inc., Santa Clara, CA, USA) as described elsewhere (Ihnen et al. 2008).

For validation purposes, RT-qPCR was applied to the total RNA isolated from identical fresh tissue biopsies as described above to validate the array data by an independent technical approach. Gene specific TaqMan-based Primer/Probe sets for the assessment of the expression of HLA-J were used. For a detailed analysis of gene expression by RT-qPCR methods, primers flanking the region of interest and a fluorescently labeled probe hybridizing in-between were utilized. Target-specific primers and probes were selected using the NCBI primer designing tool (www.ncbi.nlm.nih.go). RNA-specific primer/probe sequences were used to enable RNA-specific measurements by locating primer/probe sequences across exon/exon boundaries. Furthermore, primers/probes were selected not to bind to sequence regions with known polymorphisms (SNPs). In case multiple isoforms of the same gene existed, primers were selected to amplify all relevant or selected splice variants as appropriate. All primer pairs were checked for specificity by conventional PCR reactions. After further optimization of the primers/probes, the primers and probes listed in Table 1 gave the best results. These primers/probes are superior to primers/probes known from the prior art, e.g., in terms of specificity and amplification efficiency. To standardize the amount of sample RNA, the CALM2 was selected as reference gene, since they were not differentially regulated in the samples analyzed. Paired samples having low RNA content (i.e. Raw CT values for CALM2 of less than 22) for pretreatment biopsy or post treatment resectate were excluded.

**Table 1. Used primers and probes for HLA-J mRNA quantitation**

| Gene | Forward-Primer | Probe | Reverse-Primer |
|---|---|---|---|
| HLA-J | CCTCTGTCTCTACACCTCCATTCC | AGCTCCCTCTCTGGCACCAAGCTCC | TTCCCCTGGACTCTTCAAAAAG |
| HLA-J | CCCTCTCTGGCACCAAGCT | AGCCTGCGACGGGTCCTTCTTCCT | GGTCCGCGTCGTGAGTGT |
| HLA-J | GACCCT GCTCCGCTACTACAA | CAGAGCGAGGCGGACCCCCC | AGAACCCAGGACCTCGTTATGC |
| HLA-J | ACCCCCCCCAAGACACA | TGACCCACCCCCCTCTCTGAACATG | GAACCCAGGACCTCGTTATGC |
| HLA-J | GACCAGACCCAGGACATGGA | CTCGTGGAGACCAGGCCCACAGG | TACCACAACCGCCCACTTCT |
| HLA-J | TGCC CAAGCCCCTC ATC | TGAGATGGGTCACACATTTCTGGAAACTTCTC | CAGGGCCATGAGGTCCTAGA |
| HLA-J | TGCC CAAGCCCCTC ATC | TGAGATGGGTCACACATTTCTGGAAACTTCTC | CTAGAGGAACCTCCTAGTCTTGGAAC |
| HLA-J | GGAAACTTCTCAAGGTTCCAAGAC | AGGTTCCTCTAGGACCTCATGGCCCTGC | CTGTGAGAGGCCAGGAAGGTA |
| HLA-J | GGAGCTACTCTCAGGCTGCAA | CAGCCAAAGTGCCCAGGGCTC | CTTTACAAGCCGTGAGAGACACAT |
| HLA-J | TGCCTTGTGTGGGACTGAGA | CAAGATTTGTTCATGCCTTCCCTTTGTGAC | CAGAAAGAGAAGTCAGGGTTCGA |
| HLA-J | CTTCCCCGATCACCTTTCCT | CAGAGAAGTGGTGCTGGGATGTCTCCAT | TACAGCTCAGTGCACCATGAAGT |
| HLA-J | GCTCTCGGGGACCCTGG | ATGAGGTATTTCAGCACCGCCGTTCC | CCCACGGCAATGAAGCTG |
| HLA-J | CCAATCAGCGTCGCGCG | ACGCACCCACCGGGACTCGGAGT | ACCCCATCGTCGGCGTC |
| CALM2 | GAGCGAGCTGAGTGGTTGTG | TCGCGTCTCGGAAACCGGTAGC | AGTCAGTTGGTCAGCCATGCT |

TaqMan^{®} validation experiments were performed showing that the efficiencies of the target and the control amplifications were approximately equal, which is a prerequisite for the relative quantification of gene expression by the comparative ΔCT method. To perform the expression analysis of genes of interest within a biological sample, 4x duplex assay-mixtures were prepared by mixing the respective primers/probes of two specific assays. For separate detection of CT values, the assay probes were modified with different fluorescent probes. Each 4x assay-mix contained 2 µM of unmodified forward and reverse primers and 1.2 µM of probe. For each reaction, 2.5 µl total RNA extracted from FFPE sections (see above) were mixed with 2.5 µl assay-mix, 2.5 µl enzyme-mix and 2.5 µl water in one well of a 96-well-optical reaction plate. Measurements of the PCR reaction were done according to the instructions of the manufacturer with a Versant qPCR Cycler (Siemens) or a Light Cycler 480 (Roche) under appropriate conditions (5 min 50°C, 1 cycle; 20 s 95°C, 1 cycle; 15 s 95°C; 1 min 60°C, 40 cycles).

As depicted in Fig. 3 all ovarian cancer samples did express HLA-J. The dynamic range of HLA-J mRNA expression did comprise 5 DCT values, which means an up to 2⁵ upregulation (i.e. 30 fold) of ovarian cancer specific HLA-J expression. Importantly, this upregulation frequently occurred after neoadjuvant chemotherapy treatment in advanced ovarian cancer (compare light bars for HLA-J mRNA levels before - and dark bars for HLA-J mRNA levels after neoadjuvant chemotherapy). In 14 out of 26 (~50%) of matched sample pairs a substantial HLA-J upregulation after chemotherapy was observed, while a downregulation could only be observed for patient #107. Moreover, substantial increase of HLA-J expression coincided with a worse outcome. For example, the three patients with the highest (i.e. more than 8 fold) increase of HLA-J mRNA expression patient #106, patient #108 and patient #112 had a progression free survival interval of 8.7 months, 9.2 months and 7.9 months, respectively. Similarly, the overall survival from time between initial diagnosis and death was 10.6 months, 12.5 months and 10.3 months, respectively. In contrast, patient #107, exhibiting a marked decrease of HLA-J mRNA expression had a progression free survival interval of 15.1 months and an overall survival of 43.0 months. For comparison, the median PFS was at 14.7 months and the median OS was at 33.5 months.

Next it was analyzed whether a change in HLA-J mRNA expression has prognostic value in neoadjuvantly treated ovarian cancer patients, when looking at an > 4 fold increase of HLA-J mRNA expression in resectates after chemotherapy. Within this predefined setting 16% of the tumors did exhibit a marked increase in HLA-J expression. Indeed, the progression free survival and overall survival of such patients had been significantly worse as depicted in figures 4 and 5.

These data demonstrate that secreted forms of HLA-J are overexpressed in ovarian cancer. It further demonstrates that its expression frequently increases upon exposure of ovarian cancer with chemotherapy drugs (in more than 50% of the patients). Furthermore these data demonstrate that an extreme upregulation (more than 8 fold) is consistently associated with resistance to chemotherapy as well as reduced progression free as well as overall survival.

According to the predefined hypothesis, the investigators could prove that expression of a formerly, falsely classified "pseudogene" is of functional and prognostic value in cancer as demonstrated for ovarian cancer. The effects of neoajuvant treatment on cancer regression have long been debated. It is assumed, that the destruction of neoplastic cells results into the exposure of antigens and neoantigens to the immune system, thereby provoking a systemic mid to long term anti-tumor response ultimatively leading to prolonged survival. Still it has been unsolved, how tumors might escape these anti-tumor effects and escape from immune cell induced elimination.

Here it was show that one mechanism is the upregulation of secreted HLA-J isoforms. These small HLA-G like proteins lack the alpha 2 domain, thereby being apparently incapable of binding peptides for presentation and activation of cytotoxic and anti-neoplastic immune cells (such as T-cells). However, secretion of proteins that lack antigen presentation function, but are capable of binding to immune cells are capable of interfering with activation processes of immune cells and thereby enable tumor cell escape by inducing immune tolerance.

As the secreted isorforms are not membrane bound they built up a gradient of immunoactive ligands binding to infiltrating immune cells, which modifies their effector acitivity. In extreme cases the immune cells may be switched from anti-tumor to tumor-promoting activities. Due their small size these secreted HLA isoforms are active in a paracrine as well as distant manner. Detection of such fragments in the blood enables monitoring of anti tumor activity and respective treatment changes in case of occurring resistances. Moreover, by blocking these immunoactive ligands the effectiveness of conventional and /or other anti-neoplastic treatments including but not limited to immunotherapeutic agents (such as check point inhibitors) can be increased.

### Example 3: Determination of HLA-J mRNA expression levels by reverse transcription (RT) quantitative PCR (RT-qPCR) in a neoadjuvantly treated breast cancer (BC) patient cohort

Newly diagnosed early/locally advanced BC patients with adequate medical status for chemotherapy and breast surgery were eligible for the study. Oligometastatic BC patients were also eligible, provided that the therapeutic program included breast surgery postchemotherapy. Histologic diagnosis of BC was confirmed by core-needle biopsy. Physical examination, mammography, breast ultrasound, breast MRI, chest-abdomen CT scans, bone scans and 18F-FDG-PET/CT studies were obtained at baseline in all patients. Physical examination, mammography, breast ultrasound and 18F-FDG-PET/CT studies were repeated after two PCT (pre-operative chemotherapy) cycles and before surgery.

Patients received from 6 to 8 cycles of anthracycline based and taxane-based PCT. Surgery (including axillary lymph node dissection) was undertaken after the completion of PCT. Patients who underwent breast conserving surgery subsequently received radiotherapy.

Fresh surgical specimens (breast and axillary lymph nodes) obtained post-chemotherapy were evaluated by an experienced breast pathologist. Miller-Payne classification was the grading system used to assess histological response to PCT. Oestrogen receptor (ER), progesterone receptor (PR) and human epidermal growth factor receptor 2 (HER2) were the molecular markers examined. Immunohistochemistry (IHC) was used for the evaluation of ERs and PRs; to be positive, more than 10% of the tumor nucleus cells had to be stained. HER2 status was evaluated with IHC; HER2 positivity (HER2+ive) was defined as 3+ by IHC or 2+ IHC with gene amplification by fluorescent in situ hybridization (FISH). In addition RT-qPCR based assessment of ESR1 and ERBB2 status has been performed to obtain quantitative assessment of key parameters of breast cancer biology for determining the association of HLA gene expression with distinct breast cancer subtypes.

Sixty patients with newly diagnosed BC, whose principal characteristics are listed in Table 2, were enrolled from July 2004 to March 2011. PCT consisted of anthracycline-based and taxane based regimens for 6 cycles in nine patients (15%) and anthracycline-based and taxane-based sequential 4 cycle regimens for a total of 8 cycles in 45 patients (75%). At a median interval of 31 days (d) (range 11-52 d) from the last chemotherapy cycle all but two patients underwent surgery. After the completion of chemotherapy, an overall optimal pathologic response (pR) was observed in 13 patients (22%), and a pathologic non-response (pNR) occurred in 47 patients (78%).

**Table 2. Patient characteristics of the breast cancer cohort**

| **Patients' characteristics (n** = **60).** | n (%) |
|---|---|
| Age (median range) | 49 (31-72%) |

| **Stage n** | |
|---|---|
| (%) II A/B | 30 (50%) |
| III A/B | 23 (38%) |
| IV oligometastatic | 7 (12%) |

| **Receptor Status** | |
|---|---|
| ER+ive/HER2_ive | 31 (52%) |
| ER_ive/HER2_ive | 15 (25%) |
| HER2+ive (+3 IHC or 2+ IHC with amplified FISH) | 14 (23%) |

| **Chemotherapy** | |
|---|---|
| Anthracycline/taxane regimens (6 cycles) | 9 (15%) |
| Anthracycline/taxane-based sequential regimens (8 cy) | 45 (75%) |
| Taxane-Trastuzumab (4-8 cycles) | 6 (10%) |

| **Surgery** | |
|---|---|
| Breast-conserving surgery + axillary nodal dissection | 34 (57%) |
| Mastectomy + axillary nodal dissection | 24 (40%) |

For mRNA detection, tissues collected were snap frozen and stored in liquid nitrogen until analysis. Pretherapy core needle biopsies and post-therapy tissue resectates were crushed in liquid nitrogen. RNA was extracted using commercial kits (Qiagen), RNA integrity was assessed on the Agilent 2100 Bioanalyzer (Agilent Technologies, Palo Alto, CA, USA), cDNA was synthesized from 1 mg of total RNA using Invitrogen kits (Invitrogen Corp.) and analysed on Affymetrix HG-U133A microarrays (Affymetrix Inc., Santa Clara, CA, USA) as described elsewhere (Ihnen et al., Breast Cancer Res Treat. 2008, Volume 112, Issue 3, pp 419-427).

For validation purposes, RT-qPCR was applied to the total RNA isolated from identical fresh tissue biopsies and tumor resectates as described above to validate the array data by an independent technical approach. Gene specific TaqMan-based Primer/Probe sets for the assessment of the expression of HLA-J were used. For a detailed analysis of gene expression by RT-qPCR methods, primers flanking the region of interest and a fluorescently labeled probe hybridizing in-between were utilized. To standardize the amount of sample RNA, the CALM2, RPL37A and/or GAPDH were selected as reference genes, since they were not differentially regulated in the samples analyzed. Paired samples having low RNA content (i.e. Raw CT values for CALM2 of less than 22) for pretreatment biopsy or post treatment resectate were excluded. In total paired pre- and post-therapy samples with sufficient RNA quality were available from 19 patients. Importantly, in addition to the HLA-J RT-qPCR assays, additional RT-qPCR measurements were done to quantify subtype specific markers such as ESR1, MLPH, ERBB2, KRT5 as well as invasion markers MMP1 and MMP7 as depicted in Table 2 to assess the aggressiveness of the tumor. ESR1 status definition and subtype determination has been performed similar to as previously described (Pentheroudakis et al.. Breast Cancer Res Treat. 2008 Jul 31; Koutoula et al., Virchows Arch. 2013 Feb; 462(2):141-54, Noske et al., Breast Cancer Res Treat. 2011 Feb; 126(1): 109-17, Wirtz et al., Breast Cancer Res Treat. 2016 Jun;157(3):437-46.).

**Table 3. Used primers and probes for subtyping and determining invasive properties.**

| **GenSymbol** | **Probe_Sequenz** | **For_Sequenz** | **Rev_Sequenz** |
|---|---|---|---|
| **RPL37A** | TGGCTGGCGGTGCCTGGA | TGTGGTTCCTGCATGAAGAC A | GTGACAGCGGAAGTGGTA TTGTAC |
| **GAPDH** | AAGGTGAAGGTCGGAGTCAACGGATT TG | GCCAGCCGAGCCACATC | CCAGGCGCCCAATAG |
| **ESR1** | ATGCCCTTTTGCCGATGCA | GCCAAATTGTGTTTGATGGA TTAA | GACAAAACCGAGTCACAT CAGTAATAG |
| **ESR1** | CTGGAGATGCTGGACGCCC | CGTGGTGCCCCTCTATGAC | GGCTAGTGGGCGCATGTA G |
| **MLPH** | CCAAATGCAGACCCTTCAAGTGAGGC | TCGAGTGGCTGGGAAACTTG | AGATAGGGCACAGCCATT GC |
| **MLPH** | CGGGCGTCTTCTGAGAGTCAGATCTTT G | CGATGTGGACACCTCTGATG A | AGGCATTCCACAGCTGAA ATATG |
| **ERBB2** | AGGCCAAGTCCGCAGAAGCCCT | TCTGGACGTGCCAGTGTGAA | CCTGCTCCCTGAGGACAC |
| | | | AT |
| **ERBB2** | TGATCATGGTCAAATGTTGGATGATTG ACTC | CCATCTGCACCATTGATGTC TAC | CGGAATCTTGGCCGACAT T |
| **MMP1** | AGAGAGTACAACTTACATCGTGTTGCG GCTCA | AGATGAAAGGTGGACCAACA ATTT | CCAAGAGAATGGCCGAGT TC |
| **MMP1** | ACAGAGATGAAGTCCGGTTTTTCAAAG GGA | TTGAAGCTGCTTACGAATTT GC | GTCCCTGAACAGCCCAGT ACTTA |
| **MMP7** | CAGTCTAGGGATTAACTTCCTGTATGC T | GAACGCTGGACGGATGGTA | GAATGGCCAAGTTCATGA GTTG |
| **MMP7** | AGTGGGAACAGGCTCAGGACTATCTC AAGAG | CGGGAGGCATGAGTGAGCT A | GGCATTTTTTGTTTCTGAG TCATAGA |
| **KRT5** | TGGAGGGCGAGGAATGCAGACTCA | CGCCACTTACCGCAAGCT | ACAGAGATGTTGACTGGT CCAACTC |

The probes of Table 3 correspond to SEQ ID NOs 56 to 68, the forward primers to SEQ ID NOs 69 to 81 and the reverse primers to SEQ ID NOs 82 to 94. For instance, the probe and primers for RPL37A are SEQ ID NOs 56, 69 and 82 and for GAPDH SEQ ID NOs 57, 70 and 83. TaqMan^{®} validation experiments were performed showing that the efficiencies of the target and the control amplifications were approximately equal, which is a prerequisite for the relative quantification of gene expression by the comparative ΔCT method. To perform the expression analysis of genes of interest within a biological sample, 4x duplex assay-mixtures were prepared by mixing the respective primers/probes of two specific assays. For separate detection of CT values, the assay probes were modified with different fluorescent probes. Each 4x assay-mix contained 2 µM of unmodified forward and reverse primers and 1.2 µM of probe. For each reaction, 2.5 µl total RNA extracted from frozen tissue and formalin-fixed paraffin-embedded (FFPE) sections (see above) were mixed with 2.5 µl assay-mix, 2.5 µl enzyme-mix and 2.5 µl water in one well of a 96-well-optical reaction plate. Measurements of the PCR reaction were done according to the instructions of the manufacturer with a Versant qPCR Cycler (Siemens) or a Light Cycler 480 (Roche) under appropriate conditions (5 min 50°C, 1 cycle; 20 s 95°C, 1 cycle; 15 s 95°C; 1 min 60°C, 40 cycles).

As depicted in Fig. 6, and similar to the results in ovarian cancer (see Fig. 3) all breast cancer samples did express HLA-J. Again the dynamic range of HLA-J mRNA expression was broad comprising 7 DCT values, which means an up to 2⁷ upregulation (i.e. 128 fold) of HLA-J expression in breast cancer. Importantly, this upregulation frequently occurred after neoadjuvant chemotherapy treatment in advanced breast cancer (compare light bars for HLA-J mRNA levels before - and dark bars for HLA-J mRNA levels after neoadjuvant chemotherapy). In 10 out of 19 (~50%) of matched sample pairs a substantial HLA-J upregulation after chemotherapy was observed, while a downregulation could only be observed for patients #25 and #29.

ESR1 mRNA status was determined by predefined cut-off settings and correlated well with semiquantitative IHC determinations in this cohort. As expected, ESR1 mRNA status was inversely related to the Triple Negative Breast Cancer (TNBC) marker MMP7 (Spearman Rho 0.3656; p=0.0498), which is a matrix metalloprotease involved in WNT dependent invasion processes. In addition, ESR1 mRNA was not associated with ERBB2 mRNA overexpression, which is expected, as half of the ERBB2 positive tumors do overexpress ESR1, while the other half does not. This demonstrates the validity and representativity of the analyzed breast cancer cohort.

Non parametric Spearman correlation analysis revealed, that the HLA-J expression in core needle biopsy samples before neoadjuvant chemotherapy was significantly associated with overexpression of the ESR1 mRNA status (Spearman Rho 0.5679; p=0.0090) and trended to be significant with quantitative determination of ES1 mRNA (Spearman Rho 0.4436; p=0.0501).

Importantly, HLA-J mRNA expression before therapy also strongly correlated with KRT5 mRNA (Spearman Rho 0.6121; p=0.0041). KRT5 is thought to be associated with basal like features in breast cancer occurring in TNBC, ERBB2 and Luminal B tumors. This indicates that advanced luminal tumors harboring basal-like features predominantly express HLA-J in the chemotherapy naïve situation, indicating a de novo HLA-J expression being involved in the control of infiltrating lymphocytes.

Next the correlation of pretherapy biomarkers used for molecular subtyping with HLA-J mRNA expression in tumor tissues after neoadjuvant chemotherapy according to histopathological response of remaining tumor tissues was investigated.

Surprisingly, a complete shift of the pre-therapy associations of the subtype specific marker gene expression with regard to post therapy HLA-J mRNA expression levels was observed. By way of example, the strong and significant association of HLA-J mRNA expression in chemotherapy naïve tissues with pretherapy ESR1 mRNA status (Spearman Rho 0.5679; p=0.0090) was not only abolished, but almost reached significance in the opposite direction (Spearman Rho -0.4163; p=0.0679). Similiarly, the strong association of pre-therapy HLA-J mRNA expression with KRT5 mRNA expression (Spearman Rho 0.6121; p=0.0041) had been inverted upon chemotherapy treatment of the breast cancer tissues in vivo, while not reaching significance in this comparably small patient cohort (Spearman Rho -0.3392; p=0.01434). These findings demonstrate, that non luminal and triple negative breast cancers do dramatically upregulate HLA-J mRNA expression upon chemotherapy treatment, while the HLA-J mRNA expression does not comparably increase in luminal tumors and remains unaffected. This is of high importance as it demonstrates a subtype specific de novo expression in luminal tumors and triple negative tumors and a lack of upregulation in luminal tumors, which are known to have reduced tumor cell infiltrates (Schmidt et al., Cancer Res. 2008 Jul 1;68(13):5405-13). In contrast to these previous investigations, which focus on pre-therapy samples only, the unique approach taken hereinelucidates the dynamics of tumor driven immune cell regulation upon treatment. Importantly, the counterregulation of the tumor tissue becomes particularly evident in tumor subtypes, which do not harbor this motive de novo in the untreated setting.

Next the pretreatment levels of HLA-J expression were correlated with response to chemotherapy according to the "Tumor Regression Grade" (TRG) of Ogston et al., Breast. 2003 Oct; 12(5):320-7. This TRG system quantifies the extent of reduction in tumor size upon treatement with tumor regression grade as follows:
TRG 1: some alteration to individual malignant cells but no reduction in overall cellularity.
TRG 2: minor loss of tumor cells but overall cellularity still high; < 30% loss.
TRG 3: between 30% and 90% reduction in tumor cells.
TRG 4: > 90% loss of tumor cells.

As depicted in Figure 9, breast tumors that did not show any sign of reduction according to Miller and Pyne classification (i.e. TRG1 & TRG2; highlighted by dark gray) did simultaneously exhibit the highest HLA-J mRNA expression. In addition these tumors did have higher levels of ESR1 mRNA and lower levels of ERBB2 mRNA. This demonstrates that luminal tumors being almost unresponsive to neoadjuvant chemotherapy treatment have luminal features and highest pretreatment HLA-J mRNA levels. Therefore it is concluded that pretreatment HLA-J mRNA levels are predictive for chemotherapy response.

To compare the association between the mRNA expression of particular biomarkers and/or target genes with chemotherapy response assessment a spearman correlation has been performed using the respective continuous variables. So far ESR1 mRNA and ERBB2 are known to be some of the best response markers for predicting response of breast tumor to neoadjuvant chemotherapy in breast cancer, as has been validated in multiple large neoadjuvant trials retrospectively and prospectively (Denkert et al., Annals of Oncology 2012). Spearman correlation in the this sample set indicates that ESR1 is inversely related to tumor regression grade with high ESR1 mRNA levels being associated with low tumor regression grades. This is in line with the previous reports though not reaching statistical significance in this smaller sample set (Spearman Rho -0.2272; p=0.3495). As expected ERBB2 mRNA expression is positively correlated with tumor regression grade with high ERBB2 mRNA levels being associated with high tumor regression grades. This is in line with the previous reports though not reaching statistical significance in this smaller sample set (Spearman Rho 0.2751; p=0.2543). In contrast, HLA-J mRNA expression is inversely related with tumor regression grade with high significance (Spearman Rho -0.6094; p=0.0056). This demonstrates that pre-treatment HLA-J mRNA levels are superior to conventional breast cancer marker to predict response to neoadjuvant chemotherapy, with high levels of HLA-J in pretreatment core needle biopsies indicating non-response to chemotherapy.

Next, it was analyzed whether the post-chemotherapy mRNA levels of HLA-J mRNA in residing tumor tissues are associated with response to neoadjuvant chemotherapy.

Interestingly, the mRNA levels of breast tumors having no or minimal responses to neoaadjuvant chemotherapy revealed to have similar levels of ESR1 and HLA-J mRNA expression (compare with Figure 9). In contrast, tumors being attacked by chemotherapeutic regimen (light gray) demonstrated a marked increase in HLA-J expression. Of note, 50% of the tumors had HLA-J mRNA levels above the highest value before therapy. Simultaneously the frequency of tumors having low levels of HLA-J decreased dramatically.

It is of importance to note that the tumor regression grades 3 and 4 are associated with marked reduction though not complete reduction of tumor burden at the primary site. However, tumors that do only have subtotal or incomplete response towards chemotherapy do not have a better survival as only complete response is associated with superior progression free and overall survival.

Interestingly, when looking at post-therapy mRNA levels of biomarkers and/or target gene expression tumors having subtotal responses to chemotherapy were enriched for residing basal like tumor cells simultaneously exhibiting significant upregulation of HLA-J mRNA. Elevated post-therapy mRNA expression of KRT5 and HLA-J were associated with high TRG status (Spearman Rho 0.5239; p=0.0256 and Spearman Rho 0.5821; p=0.0089 respectively).

This finding was further investigated by scatter plot analysis of HLA-J mRNA expression based on TRG status. As displayed in Figure 13, there is a stepwise decrease of median HLA-J mRNA expression in core needle biopsies when comparing TRG2 with TRG3 and TRG4 tumors before neoadjuvant chemotherapy. In contrast, after chemotherapy there is a stepwise increase of median HLA-J mRNA expression in residual primary disease after neoadjuvant chemotherapy.

In conclusion, there is a 4 fold to 32 fold increase of HLA-J mRNA expression in non-luminal tumors having elevated immune infiltrates (as described by Schmidt et al., Clin Cancer Res 2008) upon chemotherapeutic treatment, which in addition linearly correlates with the extent of response. This can be regarded as a counter reaction of the tumor tissue to an overwhelming immune response triggered by substantial destruction of tumor cells and exposition of tumor antigens by infiltrating dendritic cells or macrophages.

The outmost predictive value of change in HLA-J mRNA expression upon chemotherapy can be displayed by subtracting pre-treatment from post-treatment HLA-J mRNA levels for individual tumor pairs (see Figure 14).

Finally, it was looked at the relevance of changes of HLA-J mRNA expression on progression free survival of breast cancer patients. Kaplan Meier analysis revealed that increase in HLA-J mRNA expression above 3,5 DDCT values indicated worse progression free survival (p=0.0096).

In summary, these results demonstrate, that pre-therapy HLA-J mRNA expression predicts therapy outcome and effectiveness of chemotherapy in breast cancer. Moreover, and similar to the situation in ovarian cancer (see Example 1) there are dynamic differences in HLA-J expression upon chemotherapeutic treatment demonstrating a counter reaction of tumor tissues to prevent elimination by increased immune infiltration upon chemotherapy induced destruction of tumor cells and subsequent recognition of the tumor lesion. Similar to the situation in ovarian cancer the extent of the counterreaction is associated with worse prognosis. Thereby a general mechanism was identified by which tumors escape therapy induced immune recognition and destruction. The determination of this mechanism was reduced to a simple, highly reproducible assay system being easily adaptable in clinical routine settings. Furthermore, tumor specific differences of this general mechanism was demonstrated by showing differences between luminal tumors as identified by way of illustration and not by limitation by determining ESR1, ERBB2 and/or KRT5 expression levels. Previously, it was shown that molecular subtyping as exemplary reduced to ESR1 mRNA determination is also of prognostic and also predictive value with regard to therapy effectiveness in all kind of tumors such as breast cancer (PCT/EP2014/067675; EP20140755642), ovarian cancer (WO2009068655A1; PCT/EP2008/066449), bladder cancer (WO 2016/131875 A1; PCT/EP 2016/053372) and lung cancer (US 9,683,229 B2; EP10721373.8). Thus, the invention illustrated by way of example and not of restriction for breast cancer is also of relevance in luminal and non-luminal basal tumors originating from other primary tumor sites due to the underlying, similar tumor biology. The inhibition of the escape mechanism disclosed herein therefore also plays an important adjunct to current therapies in diverse cancer indications.

### Example 4: Detection of the HLA-J protein via HLA-J antibodies in samples from cancer patients

Analysis of the peptide sequence homologies of the classical (HLA-A to C), non-classical (HLA-E to G) and pseudogenes (HLA-J and H) has been performed with the Geneious software in the Geneious alignment mode. The consensus sequence is highlighted in grey and above all analyzed sequences. Differences in the peptide sequences to the consensus sequence are also highlighted in grey. It can be observed, that the alpha 3 domain, the connecting peptide and its corresponding transmembrane domain of HLA-J are almost continuously highlighted in grey, showing that these regions are unique for HLA-J, compared to the pseudogene HLA-H and the classical and non-classical HLA peptide sequences. Due to this uniqueness, a HLA-J antibody has been generated against the c-terminal end of the unique alpha 3 and transmembrane domain of HLA-J (figure 16). The peptide sequence includes 22 amino acids spanning the alpha3 domain, the connecting peptide and the N-terminal end of the transmembrane domain.

In order to proof the existence of the predicted protein of HLA-J western blot analysis has been performed in ovarian cancer tissue from patients (n=4; patient ID: 107, 114, 116, 128) which did not respond to chemotherapy (figure 17 A). For each patient, HLA-J protein expression has been evaluated before (pre) and after (post) chemotherapy. 15µg of protein tissue lysates were separated in a 10% SDS-PAGE gel under denatured conditions and transferred wet to a nitrocellulose membrane. After incubation with the specific anti-HLA-J antibody "JULY", purple precipitates have been observed after incubation with an anti-rabbit antibody coupled with HPR, followed by the application of TMB substrate. Western blot analysis revealed the existence of a HLA-J protein with the observed size of approximately 55 kDa. Regarding the sample 107 post, further bands can be detected at around 100 kDa. The calculated protein size of HLA-J is around 26,7 kDa. These findings indicate that HLA-J mainly exists in a dimer and tetramer conformations, caused by cysteine residues which can create disulfide bonds. Surprisingly, within the patients 107, 116 and 128 changes in protein expression can be observed between tissues before (pre) and after (post) chemotherapy. Protein expression slightly increases from pre to post samples in patient #107 and #116. A strong increase in HLA-J protein expression can be observed from pre to post neoadjuvant treated cancer tissue in patient #128, as observed in mRNA expression analysis. This result indicates that HLA-J contributes to immune evasion, especially after neoadjuvant chemotherapy, resulting in an insufficient response to the chemotherapeutical treatment. In order to determine wether HLA-J protein is present in other types of cancers and under pre-implantation or later stages of pregnancy protein lysates from diverse cancer indications and human placenta tissue were analyzed by western blot technology similar as being described above (Figure 17 B). Importantly, HLA-J protein could be detected in ovarian cancer ("OC"), breast cancer ("BC"), urothelial/blacker cancer ("UC") and plazental tissue ("PI"). This demonstrates that HLA-J protein expression occurs under non-physiological conditions (e.g. cancer) and defined physiological situations (e.g. "pregnancy").

### Example 5: Predictive value of HLA-J expression in non-gynecological cancer

In order to evaluate whether HLA-J upregulation upon chemotherapeutic treatment also occurs in non-gynecological cancer, tissue samples from patients (both gender) suffering advanced, muscle invasive bladder cancer were collected befor chemotherapy ("TUR"; transurotheral biopsies) and after chemotherapy ("CYS"; cystectomy tissue) were collected. In addition, to proof tumor specific expression of HLA-J non-tumorous tissue were collected from the resectate of the cystectomy ("Mapping", normal tissue samples form identical patients). The initial cohort consisted of matched tissue samples from 20 patients with histologically confirmed MIBC, UICC stage II and III (cT2-3 and cN0 or cN + M0). All patients underwent 3 neoadjuvant cycles of Gemcitabine 1250mg/m² (d1; d8) Cisplatin 70mg/m² (d1) followed by radical cystectomy (RC). Representative Formalin-Fixed Paraffin-Embedded (FFPE) blocks with at least 50% tumor content (minimal tumor size 5x5 mm) were selected for the transurethral resection ("TUR") biopsies and cystectomy specimen ("CYS" samples), well delimited invasion borders, and without necrotic regions or granulomatous inflammation were selected. For the normal tissue control histopathologically confirmed, tumor-free regions from the same cystectomy specimen were selected for preparing representative FFPE blocks ("Mapping" samples). RNA was extracted from FFPE tissue using 10-µm sections which were processed by a commercially available bead-based extraction method (XTRACT kit; STRATIFYER Molecular Pathology GmbH, Cologne, Germany). RNA was eluted with 100 µl elution buffer and RNA eluates were analyzed. RT-qPCR was applied for the relative quantification of HLA-J mRNA as well as of CALM2 (housekeeping gene) expression by using gene-specific TaqMan^{®}-based assays as described above. Experiments were run on a Roche Light Cycler LC480 (Roche, Germany) according to the following protocol: 5 min at 50°C, 20 s at 95 °C followed by 40 cycles of 15 s at 95 °C, and 60 s at 60 °C. Forty amplification cycles were applied and the cycle quantification threshold (Ct) values of three markers and one reference gene for each sample were estimated as the mean of the triplicate measurements. Ct values were normalized by subtracting the Ct value of the housekeeping gene CALM2 from the Ct value of the target genes (ΔCt). HLA-J specific mRNA expression was determined by duplicate measurements using single-step RT-qPCR from matched TUR biopsies, CYS samples and mapping samples as described above. Relative gene expression was determined by using the 40-DCT Method described above.

Importantly, none of the mapping samples exhibited HLA-J expression proving the tumor specific expression of HLA-J. However, in three out of 20 patients a tumor-specific expression of HLA-J could be detected in CYS samples after neoadjuvant chemotherapy, while no expression could be detected in the matched TUR biopsy samples before chemotherapeutic treatment (Figure 18). This indicates that the tumor specific HLA-J expression had been induced by the chemotherapy contributing to the chemotherapy-resistant phenotype of these tumors. This proves that increased HLA-J expression after neoadjuvant chemotherapy not only occurs in breast and ovarian cancer, but also in non-gynecological cancers, such as bladder cancer. HLA-J expression can therefore be assumed to be a more general resistance mechanism being present in a multitude of gynecological and non-gynecological tumors.

## Claims

1. A nucleic acid molecule, a vector, a host cell, or a protein or peptide, or combinations thereof for use as an immunosuppressant medicament or as a tumor vaccine , wherein
(I) the nucleic acid molecule is
(a) encoding a polypeptide comprising or consisting of the amino acid sequence of SEQ ID NO: 1; or
(b) consisting of the nucleotide sequence of SEQ ID NO: 2; or
(c) encoding a polypeptide which is at least 80% identical, preferably at least 90% identical, and most preferred at least 95% identical to the amino acid sequence of SEQ ID NO: 1; or
(d) consisting of a nucleotide sequence which is at least 80% identical, preferably at least 90% identical, and most preferably at least 95% identical to the nucleotide sequence of SEQ ID NO: 2; or
(e) consisting of a nucleotide sequence which is degenerate with respect to the nucleic acid molecule of (d); or
(f) a fragment of the nucleic acid molecule of any one of (a) to (e), said fragment comprising at least 350 nucleotides, preferably at least 450 nucleotides, and most preferably at least 600 nucleotides; or
(g) corresponding to the nucleic acid molecule of any one of (a) to (f), wherein T is replaced by U;
(II) the vector comprises the nucleic acid molecule of (I);
(III) the host cell is transformed, transduced or transfected with the vector of (II); and
(IV) the protein or peptide being encoded by the nucleic acid molecule of (I).

2. An inhibitor of the nucleic acid molecule as defined in claim 1 and/or a binding molecule of the protein as defined in claim 1, preferably an inhibitor of the protein as defined in claim 1 for use in the treatment of a tumor, wherein
(i) the inhibitor of the nucleic acid molecule is selected from an aptamer, a siRNA, a shRNA, a miRNA, a ribozyme, an antisense nucleic acid molecule, a CRISPR-Cas9-based construct, a CRISPR-Cpf1-based construct, a meganuclease, a zinc finger nuclease, and a transcription activator-like (TAL) effector (TALE) nuclease, and/or
(ii) the binding molecule of the protein, preferably the inhibitor of the protein is selected from an antibody or antibody mimetic, and an aptamer, wherein the antibody mimetic is preferably selected from affibodies, adnectins, anticalins, DARPins, avimers, nanofitins, affilins, Kunitz domain peptides, Fynomers^{®}, trispecific binding molecules and probodies.

3. A method for diagnosing a tumor comprising detecting the presence of the nucleic acid molecule as defined in claim 1(I)(g) and/or the protein or peptide as defined in claim 1 in a sample obtained from a subject, wherein the presence of the nucleic acid molecule as defined in claim 1(I)(g) and/or the protein as defined in claim 1 is indicative for a tumor in the subject.

4. A method for grading a tumor and/or for tumor prognosis comprising determining the level of the nucleic acid molecule of as defined in claim 1(I)(g) and/or the protein or peptide as defined in claim 1 in a sample obtained from a subject, wherein increased levels of the nucleic acid molecule as defined in claim 1(I)(g) and/or the protein or peptide as defined in claim 1 as compared to a control that has been obtained from one or more subjects known to have a tumor expressing HLA-J correlate with the a higher grade of the tumor and/or an adverse tumor prognosis.

5. A method for monitoring the non-efficacy of a tumor treatment in a subject having a tumor comprising (a) determining the amount of the nucleic acid molecule as defined claim 1(I)(g) and/or the protein or peptide as defined in claim 1 in a sample obtained from a subject before the start of the treatment; and (b) determining the amount of the nucleic acid molecule as defined in claim 1(I)(g) and/or the protein or peptide as defined in claim 1 in a sample obtained from a subject at one or more times after the start of the treatment, wherein an increased amount in b) as compared to a) is indicative for the non-efficacy of a tumor treatment and/or a decreased amount in b) as compared to a) is indicative for the efficacy of a tumor treatment.

6. A method for monitoring the non-efficacy of a immunosuppressive therapy in a subject requiring such a therapy comprising (a) determining the amount of the nucleic acid molecule as defined in claim 1(I)(g) and/or the protein or peptide as defined in claim 1 in a sample obtained from a subject before the start of the therapy; and (b) determining the amount of the nucleic acid molecule as defined in claim 1(I)(g) and/or the protein or peptide as defined in claim 1 in a sample obtained from a subject at one or more times after the start of the therapy, wherein a decreased amount in b) as compared to a) is indicative for the non-efficacy of a immunosuppressive therapy and/or an increased amount in b) as compared to a) is indicative for the efficacy of a immunosuppressive therapy .

7. The nucleic acid molecule, the vector, the host cell, and/or the protein or peptide, or combinations thereof for use of claim 1, the binding molecule, preferably the inhibitor for use of claim 2, or the method of claim 3, 4 or 5, wherein the tumor is cancer.

8. The nucleic acid molecule, the vector, the host cell, and/or the protein or peptide, or combinations thereof, the binding molecule, preferably the inhibitor for use of claim 7, or the method of claim 7, wherein the cancer is selected from the group consisting of breast cancer, ovarian cancer, endometrial cancer, vaginal cancer, vulvacancer, bladder cancer, salivary gland cancer, pancreatic cancer, thyroid cancer, kidney cancer, lung cancer, cancer concerning the upper gastrointestinal tract, colon cancer, colorectal cancer, prostate cancer, squamous-cell carcinoma of the head and neck, cervical cancer, glioblastomas, malignant ascites, lymphomas and leukemias.

9. The nucleic acid molecule, the vector, the host cell, and/or the protein or peptide, or combinations thereof, the binding molecule, preferably the inhibitor for use of claim 7, or the method of claim 7, wherein the cancer is a gynecologic cancer.

10. The nucleic acid molecule, the vector, the host cell, and/or the protein or peptide, or combinations thereof, the binding molecule, preferably the inhibitor for use of claim 7, or the method claim 7, wherein the cancer is breast cancer or ovarian cancer.

11. The nucleic acid molecule, the vector, the host cell, and/or the protein or peptide, or combinations thereof, the binding molecule, preferably the inhibitor for use of any preceding claim, or the method of any preceding claim, wherein the sample is a body fluid or a tissue sample from an organ.

## Patentansprüche

1. Ein Nukleinsäuremolekül, Vektor, Wirtszelle oder Protein oder Peptid oder Kombinationen davon zur Verwendung als immunsuppressives Medikament oder als Tumorimpfstoff, wobei
(I) das Nukleinsäuremolekül
(a) ein Polypeptid kodiert, das die Aminosäuresequenz SEQ ID NO: 1 umfasst oder daraus besteht; oder
(b) aus der Nukleotidsequenz SEQ ID NO: 2 besteht; oder
(c) ein Polypeptid kodiert, das zu mindestens 80 %, vorzugsweise zu mindestens 90 % und am meisten bevorzugt zu mindestens 95 % mit der Aminosäuresequenz SEQ ID NO: 1 identisch ist; oder
(d) aus einer Nukleotidsequenz besteht, die zu mindestens 80 %, vorzugsweise zu mindestens 90 % und am meisten bevorzugt zu mindestens 95 % mit der Nukleotidsequenz SEQ ID NO: 2 identisch ist; oder
(e) aus einer Nukleotidsequenz besteht, die in Bezug auf das Nukleinsäuremolekül unter (d) degeneriert ist; oder
(f) ein Fragment des Nukleinsäuremoleküls gemäß (a) bis (e) ist, wobei dieses Fragment mindestens 350 Nukleotide, vorzugsweise mindestens 450 Nukleotide und am meisten mindestens 600 Nukleotide umfasst; oder
(g) dem Nukleinsäuremolekül aus (a) bis (f) entspricht, wobei T durch U ersetzt ist;
(II) der Vektor das Nukleinsäuremolekül aus (I) umfasst;
(III) die Wirtszelle mit dem Vektor aus (II) transformiert, transduziert oder transfiziert ist; und
(IV) das Protein oder Peptid durch das Nukleinsäuremolekül aus (I) kodiert wird.

2. Inhibitor des Nukleinsäuremoleküls nach Anspruch 1 und/oder ein Bindungsmolekül des Proteins nach Anspruch 1, vorzugsweise ein Inhibitor des Proteins nach Anspruch 1, zur Verwendung in der Bewaldung eines Tumors, wobei
(i) der Inhibitor des Nukleinsäuremoleküls ausgewählt ist aus einem Aptamer, einer siRNA, einer shRNA, einer miRNA, einem Ribozym, einem Antisense-Nukleinsäuremolekül, einem CRISPR-Cas9-basierten Konstrukt, einem CRISPR-Cpf1-basierten Konstrukt, einer Meganuklease, einer Zinkfingernuklease und einer TAL-Effektornuklease (TALE) und/oder
(ii) das Bindungsmolekül des Proteins, vorzugsweise der Inhibitor des Proteins, ausgewählt ist aus einem Antikörper oder Antikörpermimetikum und einem Aptamer, wobei das Antikörpermimetikum vorzugsweise ausgewählt ist aus Affibodies, Adnectinen, Anticalinen, DARPinen, Avimeren, Nanofitinen, Affilinen, Kunitz-Domäne-Peptiden, Fynomeren^{®}, trispezifischen Bindungsmolekülen und Probodies.

3. Verfahren zur Diagnose eines Tumors, umfassend den Nachweis des in Anspruch 1(I)(g) definierten Nukleinsäuremoleküls und/oder des in Anspruch 1 definierten Proteins oder Peptids in einer von einem Individuum gewonnenen Probe, wobei das Vorhandensein des in Anspruch 1(I)(g) definierten Nukleinsäuremoleküls und/oder des in Anspruch 1 definierten Proteins auf einen Tumor in dem Individuum hinweist.

4. Verfahren zur Graduierung eines Tumors und/oder zur Tumorprognose, umfassend die Bestimmung der Konzentration des in Anspruch 1(I)(g) definierten Nukleinsäuremoleküls und/oder des in Anspruch 1 definierten Proteins oder Peptids in einer von einem Individuum gewonnenen Probe, wobei erhöhte Konzentrationen des in Anspruch 1(I)(g) definierten Nukleinsäuremoleküls und/oder des in Anspruch 1 definierten Proteins oder Peptids im Vergleich zu einer Kontrollprobe, die von einem oder mehreren Individuen mit einem HLA-J-exprimierenden Tumor stammt, mit einem höheren Tumorgrad und/oder einer ungünstigeren Tumorprognose korrelieren.

5. Verfahren zur Überwachung der Unwirksamkeit einer Tumorbehandlung bei einem Individuum mit einem Tumor, umfassend (a) die Bestimmung der Menge des Nukleinsäuremoleküls gemäß Anspruch 1(I)(g) und/oder des Proteins oder Peptids gemäß Anspruch 1 in einer Probe, die dem Individuum vor Behandlungsbeginn entnommen wurde; und (b) die Bestimmung der Menge des Nukleinsäuremoleküls gemäß Anspruch 1(I)(g) und/oder des Proteins oder Peptids gemäß Anspruch 1 in einer Probe, die dem Individuum zu einem oder mehreren Zeitpunkten nach Behandlungsbeginn entnommen wurde, wobei eine erhöhte Menge in b) im Vergleich zu a) auf die Unwirksamkeit einer Tumorbehandlung hinweist und/oder eine verringerte Menge in b) im Vergleich zu a) auf die Wirksamkeit einer Tumorbehandlung hinweist.

6. Verfahren zur Überwachung der Unwirksamkeit einer immunsuppressiven Therapie bei einem Individuum, das eine solche Therapie benötigt, umfassend (a) die Bestimmung der Menge des Nukleinsäuremoleküls gemäß Anspruch 1(I)(g) und/oder des Proteins oder Peptids gemäß Anspruch 1 in einer Probe, die von einem Individuum vor Beginn der Therapie entnommen wurde; und (b) die Bestimmung der Menge des Nukleinsäuremoleküls gemäß Anspruch 1(I)(g) und/oder des Proteins oder Peptids gemäß Anspruch 1 in einer Probe, die von einem Individuum zu einem oder mehreren Zeitpunkten nach Beginn der Therapie entnommen wurde, wobei eine verringerte Menge in b) im Vergleich zu a) auf die Unwirksamkeit einer immunsuppressiven Therapie hinweist und/oder eine erhöhte Menge in b) im Vergleich zu a) auf die Wirksamkeit einer immunsuppressiven Therapie hinweist.

7. Nukleinsäuremolekül, Vektor, Wirtszelle und/oder Protein oder Peptid oder Kombinationen davon zur Verwendung nach Anspruch 1, Bindungsmolekül, vorzugsweise Inhibitor zur Verwendung nach Anspruch 2, oder Verfahren nach Anspruch 3, 4 oder 5, wobei der Tumor Krebs ist.

8. Nukleinsäuremolekül, Vektor, Wirtszelle und/oder Protein oder Peptid oder Kombinationen davon, Bindungsmolekül, vorzugsweise der Inhibitor zur Verwendung nach Anspruch 7, oder Verfahren nach Anspruch 7, wobei der Krebs ausgewählt ist aus der Gruppe bestehend aus Brustkrebs, Eierstockkrebs, Endometriumkarzinom, Vaginalkrebs, Vulvakrebs, Blasenkrebs, Speicheldrüsenkrebs, Bauchspeicheldrüsenkrebs, Schilddrüsenkrebs, Nierenkrebs, Lungenkrebs, Krebs des oberen Gastrointestinaltrakts, Darmkrebs, kolorektaler Krebs, Prostatakrebs, Plattenepithelkarzinom des Kopfes und Halses, Gebärmutterhalskrebs, Glioblastome, maligner Aszites, Lymphomen und Leukämien.

9. Nukleinsäuremolekül, Vektor, Wirtszelle und/oder Protein oder Peptid oder Kombinationen davon, Bindungsmolekül, vorzugsweise der Inhibitor zur Verwendung nach Anspruch 7, oder Verfahren nach Anspruch 7, wobei der Krebs ein gynäkologischer Krebs ist.

10. Nukleinsäuremolekül, Vektor, Wirtszelle und/oder Protein oder Peptid oder Kombinationen davon, Bindungsmolekül, vorzugsweise Inhibitor zur Verwendung gemäß Anspruch 7, oder Verfahren gemäß Anspruch 7, wobei der Krebs Brustkrebs oder Eierstockkrebs ist.

11. Nukleinsäuremolekül, Vektor, Wirtszelle und/oder Protein oder Peptid oder Kombinationen davon, Bindungsmolekül, vorzugsweise Inhibitor zur Verwendung gemäß einem vorhergehenden Anspruch, oder Verfahren gemäß einem vorhergehenden Anspruch, wobei es sich bei der Probe um eine Körperflüssigkeit oder eine Gewebeprobe aus einem Organ handelt.

## Revendications

1. Molécule d'acide nucléique, vecteur, cellule hôte, ou protéine ou peptide, ou combinaisons de ceux-ci, pour une utilisation dans un médicament immunodépresseur ou en tant que vaccin antitumoral, dans lesquels
(I) la molécule d'acide nucléique
(a) code un polypeptide comprenant ou consistant en la séquence d'acides aminés de la SEQ ID NO : 1 ; ou
(b) consiste en la séquence de nucléotides de la SEQ ID NO : 2 ; ou
(c) code un polypeptide qui est identique à au moins 80 %, de préférence identique à au moins 90 % et tout spécialement identique à au moins 95 % à la séquence d'acides aminés de la SEQ ID NO : 1 ; ou
(d) consiste en une séquence de nucléotides qui est identique à au moins 80 %, de préférence identique à au moins 90 % et tout spécialement identique à au moins 95 % à la séquence de nucléotides de la SEQ ID NO : 2 ; ou
(e) consiste en une séquence de nucléotides qui est dégénérée par rapport à la molécule d'acide nucléique de (d) ; ou
(f) est un fragment de la molécule d'acide nucléique de l'un quelconque de (a) à (e), ledit fragment comprenant au moins 350 nucléotides, de préférence au moins 450 nucléotides, et tout spécialement au moins 600 nucléotides ; ou
(g) correspond à la molécule d'acide nucléique de l'un quelconque de (a) à (f) dans laquelle T est remplacée par U ;
(II) le vecteur comprend la molécule d'acide nucléique de (1) ;
(III) la cellule hôte est transformée, transduite ou transfectée avec le vecteur de (II) ; et
(IV) la protéine ou le peptide est codé par la molécule d'acide nucléique de (I).

2. Inhibiteur de la molécule d'acide nucléique telle que définie dans la revendication 1 et/ou d'une molécule de liaison de la protéine telle que définie dans la revendication 1, de préférence inhibiteur de la protéine telle que définie dans la revendication 1, pour une utilisation dans le traitement d'une tumeur, dans lequel
(i) l'inhibiteur de la molécule d'acide nucléique est choisi parmi un aptamère, un ARNsi, un ARNsh, un ARNmi, un ribozyme, une molécule d'acide nucléique antisens, une construction basée sur CRISPR-Cas9, une construction basée sur CRISPR-Cpf1, une méganucléase, une nucléase à doigt de zinc, et une nucléase effectrice de type activateur de transcription (TAL) (TALE), et/ou
(ii) la molécule de liaison de la protéine, de préférence l'inhibiteur de la protéine, est choisi parmi un anticorps ou un mimétique d'anticorps, et un aptamère, parmi lesquels le mimétique d'anticorps est de préférence choisi parmi les afficorps, les adnectines, les anticalines, les DARPines, les avimères, les nanofitines, les affilines, les peptides à domaine de Kunitz, les Fynomers^{®}, les molécules de liaison trispécifiques, et les procorps.

3. Méthode pour diagnostiquer une tumeur, comprenant la détection de la présence de la molécule d'acide nucléique telle que définie dans la revendication 1(I)(g) et/ou de la protéine ou du peptide tel que défini dans la revendication 1 dans un échantillon obtenu auprès d'un sujet, dans laquelle la présence de la molécule d'acide nucléique telle que définie dans la revendication 1(I)(g) et/ou de la protéine ou du peptide tel que défini dans la revendication 1 est indicative d'une tumeur chez le sujet.

4. Méthode pour classer une tumeur et/ou pour le pronostic d'une tumeur, comprenant la détermination du niveau de la molécule d'acide nucléique telle que définie dans la revendication 1(I)(g) et/ou de la protéine ou du peptide tel que défini dans la revendication 1 dans un échantillon obtenu auprès d'un sujet, dans laquelle des niveaux de la molécule d'acide nucléique telle que définie dans la revendication 1(I)(g) et/ou de la protéine ou du peptide tel que défini dans la revendication 1 supérieurs à ceux d'un témoin qui a été obtenu auprès d'un ou plusieurs sujets connus pour avoir une tumeur exprimant HLA-J sont corrélés à un grade plus élevé de la tumeur et/ou à un pronostic mauvais de la tumeur.

5. Méthode pour surveiller la non efficacité d'un traitement antitumoral chez un sujet ayant une tumeur, comprenant (a) la détermination de la quantité de la molécule d'acide nucléique telle que définie dans la revendication 1 (I)(g) et/ou de la protéine ou du peptide tel que défini dans la revendication 1 dans un échantillon obtenu auprès du sujet avant le début du traitement ; et (b) la détermination de la quantité de la molécule d'acide nucléique telle que définie dans la revendication 1(I)(g) et/ou de la protéine ou du peptide tel que défini dans la revendication 1 dans un échantillon obtenu auprès du sujet une ou plusieurs fois après le début du traitement, dans laquelle une quantité en b) supérieure à celle en a) est indicative de la non efficacité d'un traitement antitumoral et/ou une quantité en b) inférieure à celle en a) est indicative de l'efficacité d'un traitement antitumoral.

6. Méthode pour surveiller la non efficacité d'une thérapie immunodépressive chez un sujet requérant une telle thérapie, comprenant (a) la détermination de la quantité de la molécule d'acide nucléique telle que définie dans la revendication 1(I)(g) et/ou de la protéine ou du peptide tel que défini dans la revendication 1 dans un échantillon obtenu auprès du sujet avant le début de la thérapie ; et (b) la détermination de la quantité de la molécule d'acide nucléique telle que définie dans la revendication 1(I)(g) et/ou de la protéine ou du peptide tel que défini dans la revendication 1 dans un échantillon obtenu auprès du sujet une ou plusieurs fois après le début de la thérapie, dans laquelle une quantité en b) inférieure à celle en a) est indicative de la non efficacité d'une thérapie immunodépressive et/ou une quantité en b) supérieure à celle en a) est indicative de l'efficacité d'une thérapie immunodépressive.

7. Molécule d'acide nucléique, vecteur, cellule hôte, et/ou protéine ou peptide, ou combinaisons de ceux-ci, pour une utilisation selon la revendication 1, molécule de liaison, de préférence inhibiteur pour une utilisation selon la revendication 2, ou méthode selon la revendication 3, 4 ou 5, dans lequel la tumeur est un cancer.

8. Molécule d'acide nucléique, vecteur, cellule hôte, et/ou protéine ou peptide, ou combinaisons de ceux-ci, molécule de liaison, de préférence inhibiteur pour une utilisation selon la revendication 7, ou méthode selon la revendication 7, dans lequel le cancer est choisi dans le groupe constitué par un cancer du sein, un cancer ovarien, un cancer de l'endomètre, un cancer vaginal, un cancer de la vulve, un cancer de la vessie, un cancer des glandes salivaires, un cancer du pancréas, un cancer de la thyroïde, un cancer rénal, un cancer du poumon, un cancer concernant les voies gastro-intestinales supérieures, un cancer du côlon, un cancer colorectal, un cancer de la prostate, un carcinome à cellules squameuses de la tête et du cou, un cancer du col, les glioblastomes, les ascites malignes, les lymphomes, et les leucémies.

9. Molécule d'acide nucléique, vecteur, cellule hôte, et/ou protéine ou peptide, ou combinaisons de ceux-ci, molécule de liaison, de préférence inhibiteur pour une utilisation selon la revendication 7, ou méthode selon la revendication 7, dans lequel le cancer est un cancer gynécologique.

10. Molécule d'acide nucléique, vecteur, cellule hôte, et/ou protéine ou peptide, ou combinaisons de ceux-ci, molécule de liaison, de préférence inhibiteur pour une utilisation selon la revendication 7, ou méthode selon la revendication 7, dans lequel le cancer est un cancer du sein ou un cancer ovarien.

11. Molécule d'acide nucléique, vecteur, cellule hôte, et/ou protéine ou peptide, ou combinaisons de ceux-ci, molécule de liaison, de préférence inhibiteur pour une utilisation selon l'une quelconque des revendications précédentes, ou méthode selon l'une quelconque des revendications précédentes, dans lequel l'échantillon est un fluide corporel ou un échantillon tissulaire provenant d'un organe.
